# EUROPEAN PATENT SPECIFICATION

(11) **EP 2 503 993 B1**
(45) Date of publication and mention of the grant of the patent: **13.07.2016**
(21) Application number: 10782303.1
(22) Date of filing: 25.11.2010
(51) Int. Cl.: A61K 9/16, A61K 31/4152, C07D 231/22, A61P 25/00

(54) **Hydrochloride salt of 4-[2-[[5-methyl-1-(2-naphthalenyl)-1H-pyrazol-3-yl]oxy]ethyl]morpholine**
Hydrochlorid Salz von 4-[2-[[5-Methyl-1-(2-naphthalinyl)-1H-pyrazol-3-yl]oxy]ethyl]morpholin
Sel de chlorhydrate de 4-[2-[[5-methyl-1-(2-naphthalenyl)-1H-pyrazol-3-yl]oxy]ethyl]morpholine

(30) Priority: 25.11.2009 EP 09382261; 04.02.2010 EP 10382025
(43) Date of publication of application: 03.10.2012
(73) Proprietor: Laboratorios del Dr. Esteve, S.A., 08041 Barcelona (ES)
(72) Inventor: CUBERES-ALTISENT, María Rosa, E-08172 Sant Cugat del Vallés - Barcelona (ES); SOLÀ - CARANDELL, Lluis, E-43893 Altafulla - Tarragona (ES); GARCÍA - COUCEIRO, Urko, E-48004 Bilbao - Vizcaya (ES)
(74) Representative: ABG Patentes, S.L.
(86) International application number: PCT/EP2010/068256
(87) International publication number: WO 2011/064315

(56) References cited:
- EP-A1- 2 113 501
- WO-A1-2006/021462
- LEE S ET AL: "Handbook of Pharmaceutical Salts: Properties, Selection , and Use, Chapter 8 (Large-Scale Aspects of Salt Formation: Processing of Intermediates and Final Products, Chapter 12 (Monographs on Acids and Bases)", 1 January 2002 (2002-01-01), HANDBOOK OF PHARMACEUTICAL SALTS : PROPERTIES, SELECTION, AND USE, ZÜRICH : VERL. HELVETICA CHIMICA ACTA ; WEINHEIM [U.A.] : WILEY-VCH, DE, PAGE(S) 191 - 192,211, XP002548973, ISBN: 978-3-906390-26-0 page 192 page 211 - page 213
- BERGE S M ET AL: "PHARMACEUTICAL SALTS", JOURNAL OF PHARMACEUTICAL SCIENCES, AMERICAN PHARMACEUTICAL ASSOCIATION, WASHINGTON, US, vol. 66, no. 1, 1 January 1977 (1977-01-01), pages 1-19, XP002552191, ISSN: 0022-3549, DOI: DOI:10.1002/JPS.2600660104

## Description

### FIELD OF THE INVENTION

The present invention relates to the hydrochloride salt of 4-[2-[[5-methyl-1-(2-naphthalenyl)-1 H-pyrazol-3-yl]oxy]ethyl]morpholine, to pharmaceutical compositions comprising it, and to its use in therapy and/or prophylaxis of sigma receptor associated diseases.

### BACKGROUND

The search for new therapeutic agents has been greatly aided in recent years by better understanding of the structure of proteins and other biomolecules associated with target diseases. One important class of these proteins is the sigma (σ) receptor, a cell surface receptor of the central nervous system (CNS) which may be related to the dysphoric, hallucinogenic and cardiac stimulant effects of opioids. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Snyder, S.H., Largent, B.L. J. Neuropsychiatry 1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma 2 (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad. Sci., 1996, 93:8072-8077). The existence of sigma receptors in the CNS, immune and endocrine systems have suggested a likelihood that it may serve as link between the three systems.

In view of the potential therapeutic applications of agonists or antagonists of the sigma receptor, a great effort has been directed to find selective ligands. Thus, the prior art discloses different sigma receptor ligands. 4-[2-[[5-methyl-1-(2-naphthalenyl)-1 H-pyrazol-3-yl]oxy]ethyl]morpholine is one of such promising sigma receptor ligands. The compound and its synthesis are disclosed and claimed in WO 2006/021462.

4-[2-[[5-methyl-1-(2-naphthalenyl)-1H-pyrazol-3-yl]oxy]ethyl]morpholine is a highly selective sigma-1 (σ-1) receptor antagonist. It has displayed strong analgesic activity in the treatment and prevention of chronic and acute pain, and particularly, neuropathic pain. The compound has a molecular weight 337.42 uma. The structural formula of the compound is:

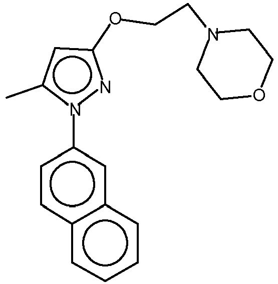

To carry out its pharmaceutical development and realize its potential, there is a need in the art for additional forms of 4-[2-[[5-methyl-1-(2-naphthalenyl)-1 H-pyrazol-3-yl]oxy]ethyl]morpholine that will facilitate the preparation of better formulations of this active pharmaceutical ingredient. Furthermore, new forms of the compound may also improve its production, handling and storage characteristics and its therapeutic effects such as pharmacological properties.

In this regard, alternative forms of the compound may have widely different properties such as, for example, enhanced thermodynamic stability, higher purity or improved bioavailability (e.g. better absorption, dissolution patterns). Specific compound forms could also facilitate the manufacturing (e.g. enhanced flowability), handling and storage (e.g. non-hygroscopic, long shelf life) of the compound formulations or allow the use of a lower dose of the therapeutic agent, thus decreasing its potential side effects. Thus it is important to provide such forms, having desirable properties for pharmaceutical use.

### BRIEF DESCRIPTION OF THE INVENTION

The inventors of the present invention, after an extensive research on different forms of 4-[2-[[5-methyl-1-(2-naphthalenyl)-1 H-pyrazol-3-yl]oxy]ethyl]morpholine (herein referred as "compound 63"), have surprisingly found and demonstrated that its hydrochloride salt provides advantageous production, handling, storage and/or therapeutic properties.

Thus, in a first aspect the present invention is directed to the hydrochloride salt of 4-[2-[[5-methyl-1-(2-naphthalenyl)-1 H-pyrazol-3-yl]oxy]ethyl]morpholine (herein referred as "P027" or "example 1 ").

Other salts of the compound 63 selected from the group consisting of ethanesulfonate, fumarate, malate, maleate, malonate and methanesulfonate are disclosed for reference purpose.

The P027 compound has a molecular weight 373.88 uma, a pKa of 6.73 and a melting point of 194.2 °C. The compound is very soluble in water and freely soluble in methanol, 1N hydrochloric acid and dimethyl sulphoxide. It is sparingly soluble in ethanol, slightly soluble in acetone and practically insoluble in ethyl acetate and in 1 N sodium hydroxide. The product exhibits a better dissolution and absorption profile in vivo than its related base.

In another aspect, the present invention is directed to a process for the preparation of the hydrochloride salt of 4-[2-[[5-methyl-1-(2-naphthalenyl)-1 H-pyrazol-3-yl]oxy]ethyl]morpholine which comprises:
a) mixing 4-[2-[[5-methyl-1-(2-naphthalenyl)-1H-pyrazol-3-yl]oxy]ethyl]morpholine and a solution containing hydrochloric acid, and
b) isolating the resulting hydrochloride salt.

A further aspect of the present invention includes pharmaceutical compositions comprising 4-[2-[[5-methyl-1-(2-naphthalenyl)-1 H-pyrazol-3-yl]oxy]ethyl]morpholine hydrochloride and a pharmaceutically acceptable carrier, adjuvant or vehicle.

In a further aspect the invention is directed to 4-[2-[[5-methyl-1-(2-naphthalenyl)-1 H-pyrazol-3-yl]oxy]ethyl]morpholine hydrochloride for use as medicament, preferably as sigma ligand, i.e., for use the treatment and/or prophylaxis of a sigma receptor mediated disease or condition.

These aspects and preferred embodiments thereof are additionally also defined in the claims.

### BRIEF DESCRIPTION OF THE FIGURES

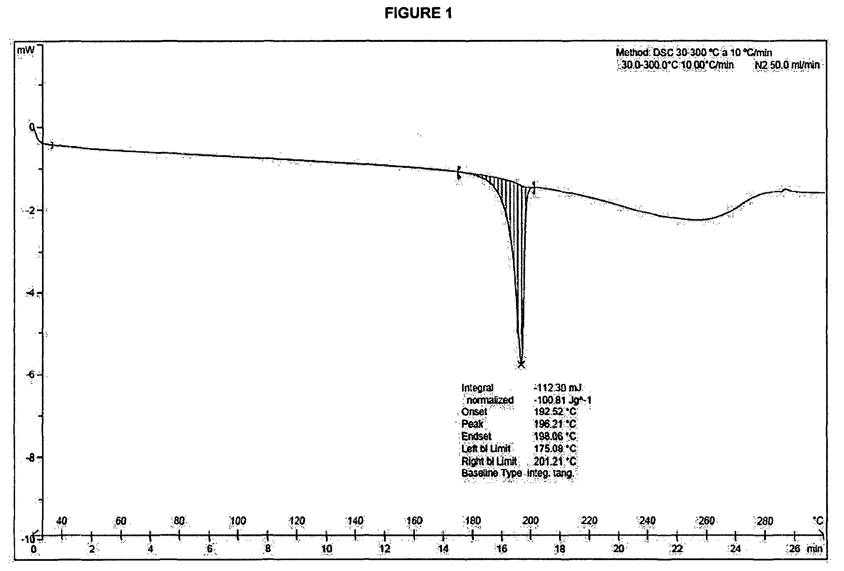

**Figure n° 1:** differential scanning calorimetry (DSC) of example 1

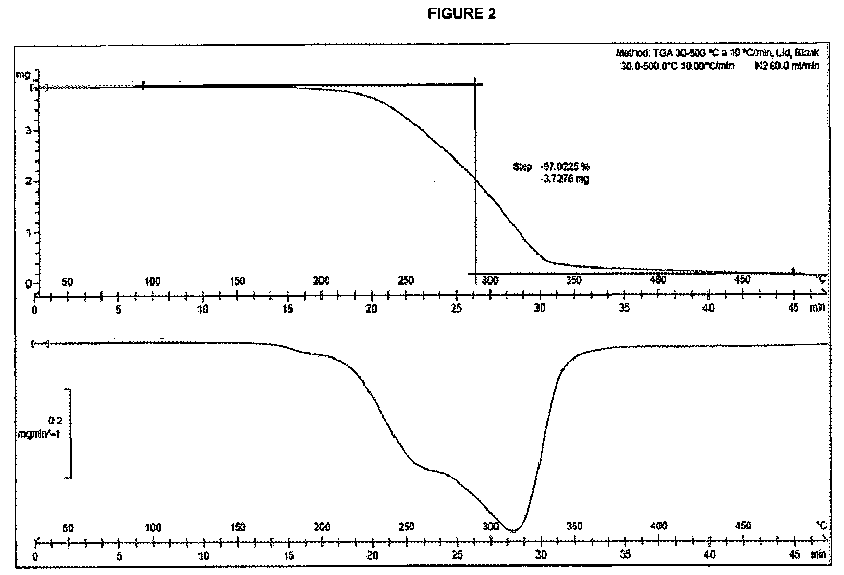

**Figure n° 2:** thermogravimetry (TGA) of example 1

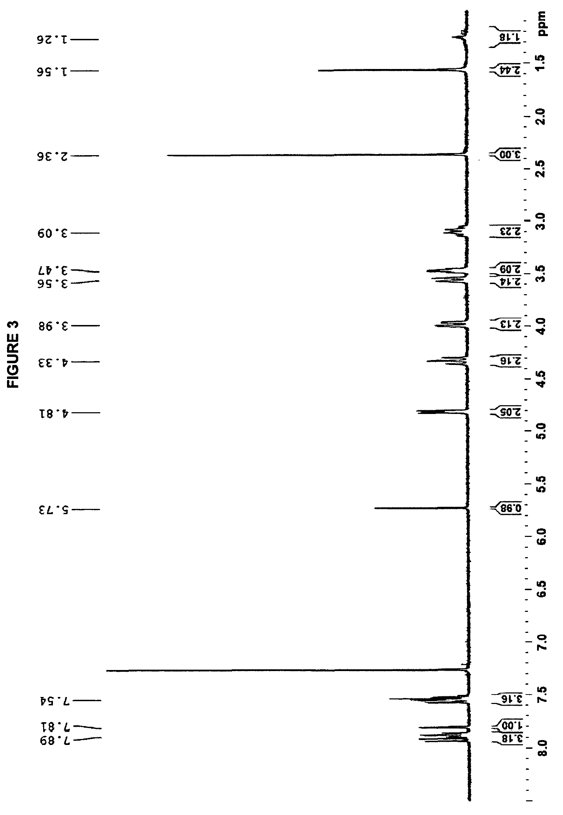

**Figure n° 3:** proton nuclear magnetic resonance (¹HNMR) of example 1

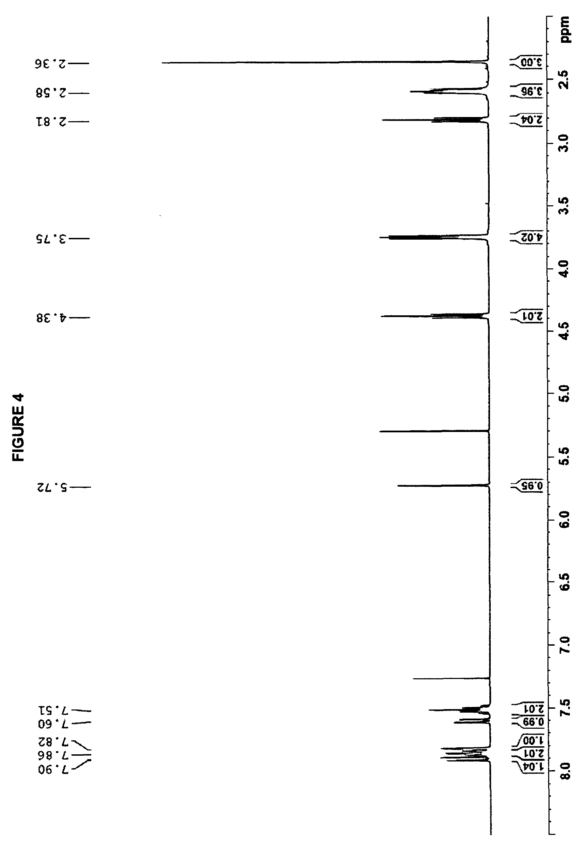

**Figure n° 4:** proton nuclear magnetic resonance (¹HNMR) of compound 63
**Figure n° 5:** proton nuclear magnetic resonance (¹HNMR) of example 2
**Figure n° 6:** differential scanning calorimetry (DSC) of example 2
**Figure n° 7:** thermogravimetry (TGA) of example 2
**Figure n° 8:** FTIR analysis of example 2
**Figure n° 9** proton nuclear magnetic resonance (¹HNMR) of example 3
**Figure n° 10:** differential scanning calorimetry (DSC) of example 3
**Figure n° 11:** thermogravimetry (TGA) of example 3
**Figure n° 12:** FTIR analysis of example 3
**Figure n° 13** proton nuclear magnetic resonance (¹HNMR) of example 4
**Figure n° 14:** differential scanning calorimetry (DSC) of example 4
**Figure n° 15:** thermogravimetry (TGA) of example 4
**Figure n° 16:** FTIR analysis of example 4
**Figure n° 17** proton nuclear magnetic resonance (¹HNMR) of example 5
**Figure n° 18:** differential scanning calorimetry (DSC) of example 5
**Figure n° 19:** thermogravimetry (TGA) of example 5
**Figure n° 20:** FTIR analysis of example 5
**Figure n° 21:** proton nuclear magnetic resonance (¹HNMR) of example 6
**Figure n° 22:** differential scanning calorimetry (DSC) of example 6
**Figure n° 23:** thermogravimetry (TGA) of example 6
**Figure n° 24:** FTIR analysis of example 6
**Figure n° 25:** proton nuclear magnetic resonance (¹HNMR) of example 7
**Figure n° 26:** differential scanning calorimetry (DSC) of example 7
**Figure n° 27:** thermogravimetry (TGA) of example 7
**Figure n° 28:** FTIR analysis of example 7
**Figure n° 29:** Thermodynamic solubility for example 1. Calibration curve.
**Figure n° 30:** Plasma concentration of Example 1 in rat

### DETAILED DESCRIPTION OF THE INVENTION

The inventors have found that the compound P027, which is the HCl salt of 4-[2-[[5-methyl-1-(2-naphthalenyl)-1 H-pyrazol-3-yl]oxy]ethyl]morpholine, has advantages due to the fact, among others, that it is a crystalline solid, which simplifies isolation, purification and handling.

Indeed, after an extensive screening of salts, the inventors have observed that a large number of acids (e.g. sulphuric acid or L-tartaric acid) did not afford a solid when mixing with the 4-[2-[[5-methyl-1-(2-naphthalenyl)-1 H-pyrazol-3-yl]oxy]ethyl]morpholine but an oil. Further, among the acids suitable for obtaining a salt in solid form, hydrochloric acid was the one that provided better results in terms of easiness of preparation, physical stability, scaling-up, solubility, etc.

Thus, the present invention relates to 4-[2-[[5-methyl-1-(2-naphthalenyl)-1H-pyrazol-3-yl]oxy]ethyl]morpholine hydrochloride (P027).

The hydrochloride salt of 4-[2-[[5-methyl-1-(2-naphthalenyl)-1 H-pyrazol-3-yl]oxy]ethyl]morpholine can be prepared by adding an hydrochloric acid solution to its corresponding base dissolved in the appropriate solvent. In a particular embodiment, the P027 compound may be conveniently obtained by dissolving the free base compound in ethanol saturated with HCl.

As noted previously, it has been reported that 4-[2-[[5-methyl-1-(2-naphthalenyl)-1 H-pyrazol-3-yl]oxy]ethyl]morpholine is a highly selective sigma-1 (σ-1) receptor antagonist, displaying strong analgesic activity in the treatment and prevention of chronic and acute pain, and particularly, neuropathic pain (see WO 2006/021462). It has now been found that the hydrochloride salt of 4-[2-[[5-methyl-1-(2-naphthalenyl)-1 H-pyrazol-3-yl]oxy]ethyl]morpholine is particularly suitable for use as medicament. The present invention therefore further provides medicaments or pharmaceutical compositions comprising 4-[2-[[5-methyl-1-(2-naphthalenyl)-1 H-pyrazol-3-yl]oxy]ethyl]morpholine hydrochloride together with a pharmaceutically acceptable carrier, adjuvant, or vehicle, for administration to a patient.

More particularly, the P027 compound is useful in the treatment and/or prophylaxis of a sigma receptor mediated disease or condition.

In a more preferred embodiment the P027 compound is used in the manufacture of a medicament for the treatment and/or prophylaxis of a disease selected from the group consisting of diarrhoea; lipoprotein disorders; migraine; obesity; arthritis; hypertension; arrhythmia; ulcer; learning, memory and attention deficits; cognition disorders; neurodegenerative diseases; demyelinating diseases; addiction to drugs and chemical substances including cocaine, amphetamine, ethanol and nicotine; tardive diskinesia; ischemic stroke; epilepsy; stroke; stress; cancer; psychotic conditions, in particular depression, anxiety or schizophrenia; inflammation; or autoimmune diseases.

The auxiliary materials or additives of a pharmaceutical composition according to the present invention can be selected among carriers, excipients, support materials, lubricants, fillers, solvents, diluents, colorants, flavour conditioners such as sugars, antioxidants, binders, adhesives, disintegrants, anti-adherents, glidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition.

The medicament or pharmaceutical composition according to the present invention may be in any form suitable for the application to humans and/or animals, preferably humans including infants, children and adults and can be produced by standard procedures known to those skilled in the art. Therefore, the formulation in accordance with the invention may be adapted for topical or systemic application, particularly for dermal, transdermal, subcutaneous, intramuscular, intra-articular, intraperitoneal, intravenous, intra-arterial, intravesical, intraosseous, intracavernosal, pulmonary, buccal, sublingual, ocular, intravitreal, intranasal, percutaneous, rectal, vaginal, oral, epidural, intrathecal, intraventricular, intracerebral, intracerebroventricular, intracisternal, intraspinal, perispinal, intracranial, delivery via needles or catheters with or without pump devices, or other application routes.

The mentioned formulations will be prepared using standard methods such as those described or referred to in the Spanish and US Pharmacopoeias and similar reference texts.

In one embodiment of the invention it is preferred that the P027 compound is used in therapeutically effective amounts. The physician will determine the dosage of the present therapeutic agent which will be most suitable and it will vary with the form of administration and the particular compound chosen, and furthermore, it will vary with the patient under treatment, the age of the patient, the type of disease or condition being treated. When the composition is administered orally, larger quantities of the active agent will be required to produce the same effect as a smaller quantity given parenterally. The compound is useful in the same manner as comparable therapeutic agents and the dosage level is of the same order of magnitude as is generally employed with these other therapeutic agents. This active compound will typically be administered once or more times a day for example 1, 2, 3 or 4 times daily, with typical total daily doses in the range of from 0.1 to 1000 mg/kg/day.

The following examples are merely illustrative of certain embodiments of the invention and cannot be considered as restricting it in any way.

### EXAMPLES

### Analytical Techniques

The following techniques have been used in this invention for identifying the different salts of compound 63 obtained:
- Differential Scanning Calorimetry analysis (DSC) DSC analyses were recorded in a Mettler Toledo DSC822e. Samples of 1-2 mg were weighted into 40 µL aluminium crucibles with a pinhole lid, and were heated, under nitrogen (50 mL/min), from 30 to 300 °C at a heating rate of 10 °C/min. Data collection and evaluation were done with software STARe.
- Thermogravimetric analysis (TGA) Thermogravimetric analyses were recorded in a Mettler Toledo SDTA851e. Samples of 3 - 4 mg were weighted (using a microscale MX5, Mettler) into open 40 µL aluminium crucibles, and heated at 10 °C/min between 30 and 300 °C, under nitrogen (80 mL/min). Data collection and evaluation were done with software STARe.
- Proton Nuclear Magnetic Resonance (¹H-NMR) Proton nuclear magnetic resonance analyses were recorded in deuterated chloroform or methanol in a Bruker Avance 400 Ultrashield NMR spectrometer, equipped with a z-gradient 5 mm BBO (Broadband Observe) probe with ATM and an automatic BACS-120 autosampler. Spectra were acquired solving 2-10 mg of sample in 0.7 mL of deuterated solvent.
- Fourier Transformed Infrared spectroscopy (FTIR) The FTIR spectra were recorded using a Bruker Tensor 27, equipped with a MKII golden gate single reflection ATR system, a mid-infrared source as the excitation source and a DTGS detector. The spectra were acquired in 32 scans at a resolution of 4 cm⁻¹. No sample preparation was required to perform the analysis.

### Example 1. Synthesis of 4-{2-[5-Methyl-1-(naphthalen-2-yl)-1H-pyrazol-3-yloxy]ethyl} morpholine (compound 63) and its hydrochloride salt (example 1)

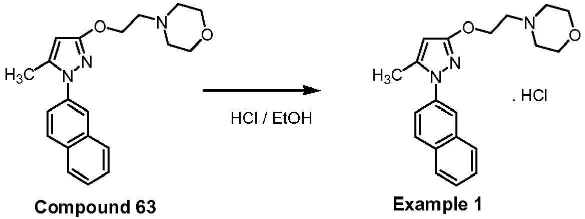

Compound 63 can be can be prepared as disclosed in the previous application WO2006/021462. Its hydrochloride can be obtained according the following procedure: Compound 63 (6,39 g) was dissolved in ethanol saturated with HCl, the mixture was stirred then for some minutes and evaporated to dryness. The residue was crystallized from isopropanol. The mother liquors from the first crystallization afforded a second crystallization by concentrating. Both crystallizations taken together yielded 5.24 g (63 %) of the corresponding hydrochloride salt (m.p. = 197-199 °C).
¹H-NMR (DMSO-d₆) δ ppm: 10,85 (bs, 1 H), 7,95 (m, 4H), 7,7 (dd, J=2,2, 8,8 Hz, 1 H), 7,55 (m, 2H), 5,9 (s, 1 H), 4,55 (m, 2H), 3,95 (m, 2H), 3,75 (m, 2H), 3,55-3,4 (m, 4H), 3,2 (m, 2H), 2,35 (s, 3H).
HPLC purity: 99.8%.

With this method, the hydrochloride salt is obtained as a crystalline solid with a very good yield. Further, its high melting point is particularly convenient from a pharmaceutical standpoint since it implies that the product shows a good physical stability.

### Extraction of compound 63 from its hydrochloride salt (example 1)

The sample used in this invention is the Example 1. The base (compound 63) was extracted with CH₂Cl₂ from a basic aqueous solution (pH > 10, using a 0.5 M aqueous solution of NaOH) of example 1, rendering orange oil.

### General method to crystallize other salts of compound 63

Salts were prepared initially mixing 1 mL of a 0.107 M solution of compound 63, as the orange oil previously obtained (see Example 1), in methanol with 1 mL of a 0.107 M solution of the corresponding counterion in methanol. The mixtures were stirred for one hour and the solvent evaporated under vacuum (Genevac, 8 mm Hg), obtaining oil or a white solid depending on the salt.

The product obtained in the initial preparation was solved in the minimum amount of crystallization solvent at its boiling temperature or at a maximum of 75 °C. If after the addition of 4 mL of solvent, the salt did not dissolve completely, the suspension was stirred at high temperature for 30 minutes and the residue was separated by hot filtration or centrifugation. The mother liquors were cooled to room temperature and kept for 24 hours.

When solid was formed, it was separated (filtration or centrifugation). If not, the solution was kept in the refrigerator (4 °C) for a few days. If solid was formed, it was separated from the solution. If not, the solution was kept in the freezer (-21 °C) for a few days. If solid was formed, it was separated from the solution. In case that after all these manipulations no solid was obtained the solution was left evaporating up to dryness.

All obtained solids were dried in the vacuum drying oven at 40 °C (10 mm Hg) for 4 hours and, if enough quantity was available, were analysed. The initial characterisation was done by ¹H-NMR to confirm the synthesis of the salt. The solvents used in this invention are listed in table 1.

**Table 1. Solvents used in this invention**

| **Name** | **Code** | **Boiling temperature (°C)** | **Melting point (°C)** | **Dielectric constant** |
|---|---|---|---|---|
| Acetone | ACE | 56 | -94 | 20,7 |
| Acetonitrile | ACN | 81 | -46 | 38,8 |
| Ethyl acetate | AET | 77 | -84 | 6 |
| Chloroform | CLF | 61 | -63 | 4,8 |
| *N,N-*Dimethylformamide | DMF | 153 | -98 | 36,7 |
| Ethanol | EOH | 78 | -114 | 24,6 |
| Isopropanol | IPH | 82 | -90 | 19,9 |
| Methanol | MOH | 65 | -98 | 32,7 |
| Tetrahydrofurane | THF | 66 | -108 | 20,4 |
| Dimethyl carbonate | CDM | 90 | 3 | 3,1 |
| Water | H2O | 100 | 0 | 80 |
| 2-Butanol | BUL | 98 | -115 | 16,6 |
| Methyl *tert*-butyl ether | MTE | 55 | -109 | 2,6 |
| Diisopropyl ether | DIE | 68 | -86 | 3,9 |
| Isobutyl acetate | AIB | 117 | -99 | 5 |
| Chlorobencene | CLB | 132 | -45 | 5,6 |
| Cyclohexane | CHE | 81 | 6 | 2,2 |
| 3-Pentanone | POA | 102 | -40 | 17 |
| Toluene | TOL | 110 | -93 | 7,6 |

The acids used to investigate the crystalline salts of compound 63 were selected according to the following criteria (Table 2):
- Acids with a pKa at least three units lower than compound 63 (pKa of 6.7)
- Acids that are pharmaceutically acceptable compounds

Although several of the acids selected have two or even three (citric acid) acidic positions, in principle, only sulfuric acid has a second proton acidic enough to form the disalt with compound 63. So in total there are eleven different salts that could be formed.

**Table 2. Selected acids used as counterions.**

| **acid** | **code** | **Purity (%)** | **pKa₁** | **pKa₂** | **pK₃** |
|---|---|---|---|---|---|
| Sulfuric acid | SFT | 95-97 | -3 | 1.9 | - |
| Methanesulfonic acid | MSF | 99.5 | -1.2 | - | - |
| Ethanesulfonic acid | ESF | 95.0 | 2.05 | - | - |
| Fumaric acid | FMT | 99.5 | 3.03 | 4.38 | - |
| L-(-)-Malic acid | LML | 99.5 | 3.46 | 5.10 | - |
| Malonic acid | MLO | 99.0 | 2.83 | 5.70 | - |
| Maleic acid | MLE | 99.0 | 1.92 | 6.23 | - |
| Citric acid | CTR | 99.5 | 3.13 | 4.76 | 6.40 |
| Glycolic acid | GLY | 99.0 | 3.82 | - | - |
| L-(+)-Tartaric acid | LTT | 99.5 | 3.02 | 4.36 | - |

The general strategy performed to study the crystalline salts of compound 63 can be divided into three steps:
- Step 1: Salt crystallization screening
- Step 2: Salt optimization and characterization
- Step 3: Large scale preparation of selected salts

Initially, a crystallization screening was performed using the selected counterions shown in Table 2, to seek for promising crystalline salts. The screening was performed at a small scale (40 mg of compound 63), using a large range of crystallization solvents (Table 1) and different crystallization methodologies. In the screening, crystallization conditions were not strictly monitored, and the solids obtained were characterized by ¹H-NMR. NMR spectroscopy gives a good indication of salt formation, since the ¹H-NMR spectrum of the salt differs substantially from that of the acid and base mixture. A clear shift of the signals associated to the hydrogens close to the protonated nitrogen is observed. Moreover, when the acid counterion has characteristic signals in the ¹H-NMR, these can be identified, allowing to determine the salt stoichiometry and to have a qualitative idea of the salt purity.

In a second step, all crystalline salts were scaled-up at 100-500 mg scale in the solvents that gave the best result in the screening procedure. Moreover, a crystallization methodology appropriate for industrial production was used. The salts obtained were fully characterized by ¹H-NMR, DSC, TGA and FTIR. The aim of this step was, first to design a scalable procedure to prepare the selected salts with an optimized yield, and second to fully characterize them.

Finally, a group of selected crystalline salts, with adequate solid state properties (crystallinity and thermal stability) were prepared at a scale of 2-3 g starting from compound 63.

### From salt crystallization screening to large scale preparation (steps 1-3)

Initially, a crystallization screening of compound 63 with the ten counterions depicted in table 2 was performed, at a 40 mg scale, in the following ten solvents: acetone, ethyl acetate, chloroform, *N*,*N*-dimethylformamide, methanol, ethanol, isopropanol, 2-butanol, acetonitrile and tetrahydrofuran. The procedure started with the preparation of equimolar mixtures, from known concentration methanol dissolutions, of compound 63 and the different acid counterions. The resulting crude, after the methanol evaporation, was crystallized from the hot solvents formerly mentioned. Different crystallization strategies were used depending on the solubility of each acid and compound 63 mixture, and therefore the solids were obtained using different procedures. For some acids, the mixture was not soluble in the hot crystallization solvent, obtaining a slurry solid. In other cases, the solid crystallized during room temperature cooling of the solution, or after several days at 4 °C or at -18 °C. Finally, in some crystallization attempts, the solid was obtained after slow evaporation of the solvent at room temperature. In several cases, more than one solid per crystallization attempt were obtained.

From this first crystallization screening (table 3), the following observations could be drawn:
- Crystalline salts of compound 63 with fumaric and maleic acids were obtained in most of the solvents assayed. For both acid counterions, several crystalline solids including solvates were obtained. All solids corresponded to the equimolecular salt.
- The equimolar mixture of compound 63 and citric acid was very soluble in the vast majority of solvents assay. Therefore, most of the solids were obtained after complete evaporation of the solvent. Moreover, the solids obtained were of low crystallinity or contained appreciable amounts of residual solvents. Most probably, the low crystalline solids came from desolvated solvates.
- The equimolar mixture of compound 63 and glycolic acid was very soluble in the vast majority of solvents assay. Therefore, most of the solids were obtained after complete evaporation of the solvent, and several were mixtures of solids.
- Crystalline salts of compound 63 with ethanesulfonic, L-malic and malonic acids were obtained only in one or two of the solvents assayed under very concentrated conditions. Most of the solids were obtained after complete evaporation of the solvent.
- No crystalline solids of compound 63 with sulfuric, methanesulfonic and L-tartaric acids were obtained. The base and acid mixtures were very soluble in all
solvents assayed and either oils or a non-crystalline solid were obtained after complete evaporation of the solvent.

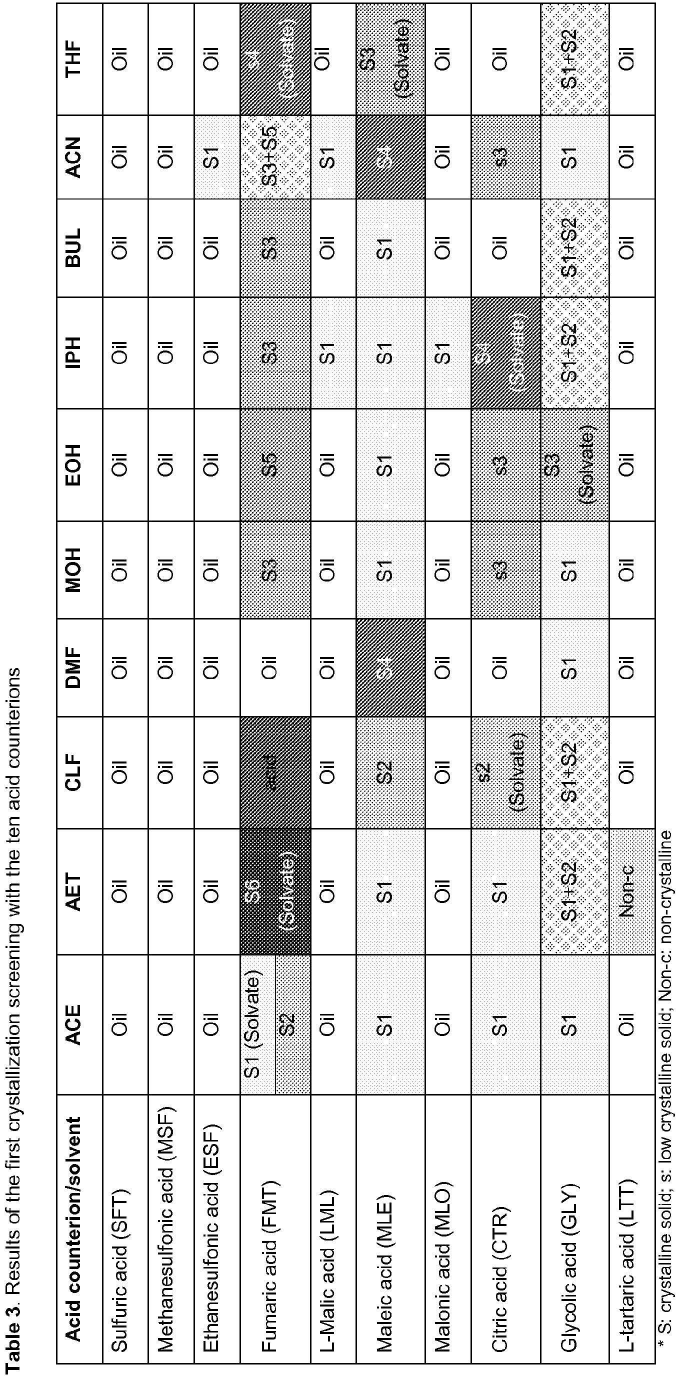

Taking into account these results, a second crystallization screening was performed in nine additional solvents. Less polar solvents (isobutyl acetate, dimethyl carbonate, chlorobenzene, cyclohexane, 3-pentanone, toluene, methyl *tert-butyl* ether, diisopropyl ether) and water were selected in order to decrease the solubility of the salts (Table 4).

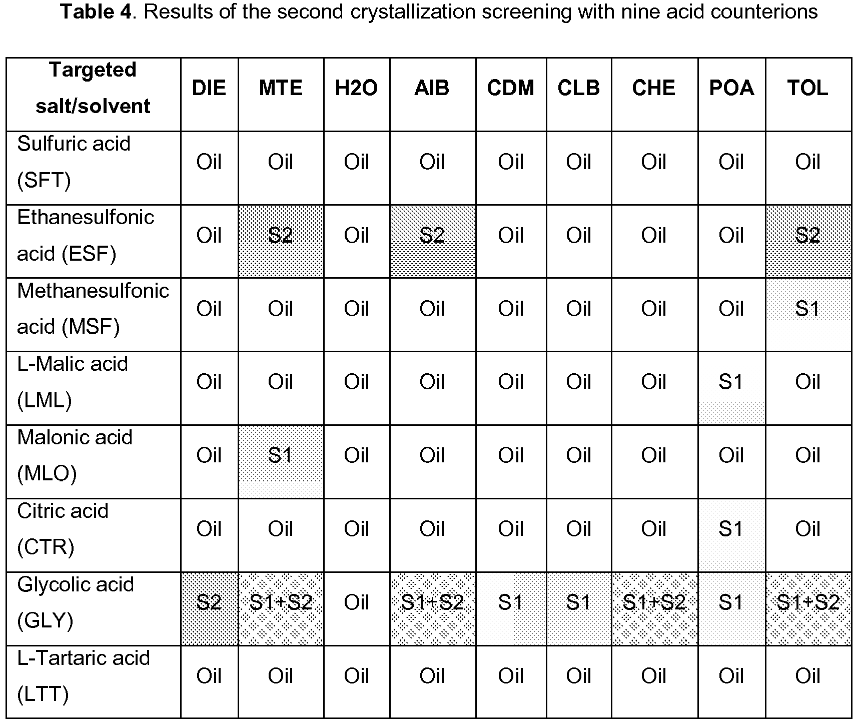

From this second crystallization screening, the following observations could be drawn:
- Although the equimolar mixture of compound 63 and glycolic acid was less soluble in this second set of solvents, the behavior was very similar to the first set of crystallizations. Several solids corresponding to mixtures of solids were obtained. Solid 1 was only generated after complete evaporation of the solvent and could not be completely characterized.
- Crystalline salts of compound 63 with L-malic, malonic and citric acids were obtained only in one solvent, rendering an already known solid.
- Crystalline salts of compound 63 with ethanesulfonic acid were obtained in several solvents, rendering, in all cases, a new solid different from the initial crystallization screening.
- A solid corresponding to a crystalline salt of compound 63 with methanesulfonic acid could be obtained in toluene.
- No crystalline solids of compound 63 with sulfuric and L-tartaric acids were obtained in this second set of solvents.

Taking into account the results of the two crystallization screenings described, we optimize the generation of the best characterized non solvated salts of compound 63 with fumaric, maleic, methanesulfonic, ethanesulfonic, L-malic, and malonic acids. The optimization scale-up experiments were performed starting from 100 mg of compound 63. The scale-up procedure was also optimized for the salts with fumaric, maleic, methanesulfonic, ethanesulfonic, L-malic and malonic acids.

Finally, the preparation of the salts for the six selected counterions was scale-up at 2-3 g and they were fully characterized. The overall process in this invention is summarized in the following table.

**Table 5. Summary of crystallizations performed with crystalline salts of compound 63.**

| ***Crystallization screening*** | ***190 crystallizations*** |
|---|---|
| Sulfuric acid, methanesulfonic acid, ethanesulfonic acid, fumaric acid, L-(-)-malic acid, malonic acid, maleic acid, citric acid, glycolic acid, L-(+)-tartaric acid | 40 mg scale |

| ***Crystalline solid optimization and characterization*** | ***23 crystallizations*** |
|---|---|
| Methanesulfonic acid, ethanesulfonic acid, fumaric acid, L-(-)-malic acid, malonic acid, maleic acid | 100-500 mg scale |

| ***Large scale preparation of selected salts*** | ***6 crystallizations*** |
|---|---|
| Methanesulfonic acid, ethanesulfonic acid, fumaric acid, L-(-)-malic acid, malonic acid, maleic acid | 2.5 g scale |

### Example 2: preparation of the fumarate salt of compound 63 (reference)

During the initial screening the crystallization of the fumarate salt was attempted in 10 different solvents. Crystalline solids corresponding to the salt were obtained in all solvents, except DMF and chloroform, using different crystallization techniques: slurry, cooling a saturated solution or after complete evaporation of the solvent. In chloroform the initial acid was recovered, whereas in DMF the salt separated as orange oil. Two non-solvated solids were obtained, the first one in methanol, isopropanol and butanol, and the second one only in ethanol. Finally, solvates were obtained in acetone, ethyl acetate and THF, and a mixture of the two solids was generated in acetonitrile.

A non-solvated crystalline solid, in principle any of the ones obtained in the screening, was chosen for the scale-up. Initially, the scale up was attempted in acetonitrile, since it was the solvent that rendered a crystalline product in which the salt was less soluble. Although the salt was obtained in very good yield (83%), the process was not optimal for scale-up since the acid is not soluble in acetonitrile and the final salt precipitated from a mixture of compound 63 as an oil and fumaric acid as a solid, both suspended in the solvent. The crystallization was then attempted in ethanol to generate pure solid S5. Very disappointingly, in the scale-up in ethanol, a new, poorly crystalline solid was generated in low yield. Finally, the crystallization was performed in acetonitrile, adding the acid dissolved in an alcohol (ethanol or isopropanol). Slightly better results are obtained when fumaric acid is dissolved in ethanol and the addition is performed at room temperature (Table 6). On the other hand, a mixture of phases was obtained when the suspension was kept at 4 °C for two days (Table 6, entry 4).

**Table 6: Experiments to scale-up the fumarate salt of compound 63**

| **Entry** | **Scale¹** | **Solvent 1²** | **Solvent 2³** | **T₁(°C)⁴/T₂(°C)⁵** | **Yield (%)⁶** |
|---|---|---|---|---|---|
| 1 | 200 mg | 2 mL ACN | 0.8 mL EtOH | 70/25 | 49 |
| 2 | 500 mg | 5 mL ACN | 2 mL EtOH | 25/25 | 59 |
| 3 | 200 mg | 2 mL ACN | 1 mL IPH | 25/25 | 55 |
| 4 | 2.5 g | 20 mL ACN | 10 mL EtOH | 25/4 | 58 |

| | | | | | |
|---|---|---|---|---|---|
| 1-) Referred to starting example 1. 2-) Solvent used to dissolve compound 63. 3-) Solvent used to dissolve the fumaric acid. 4-) Temperature at which the acid and base are mixed. 5-) Temperature at which the final solid is harvested. 6-) All experiments were seeded. | | | | | |

The experimental procedure used to prepare the fumarate salt at 0.5 g scale (entry 2 in table 6) was as follows:
A *solution of fumaric acid* (*153 mg, 1.32 mmol) in 2 mL of ethanol is added slowly to a solution of compound 63 (456 mg, 1.35 mmol) in 5 mL* of *acetonitrile at room temperature. The resulting yellow solution is seeded and is stirred at room temperature for 15 minutes. An abundant white solid precipitates readily. The resulting suspension is stirred at room temperature for 15 hours. The solid obtained is filtered off, washed with 1 mL of acetonitrile and dried under vacuum (10 mm Hg) at 45 °C for 6 hours to give the fumarate salt as a white solid (350 mg, 59%).*

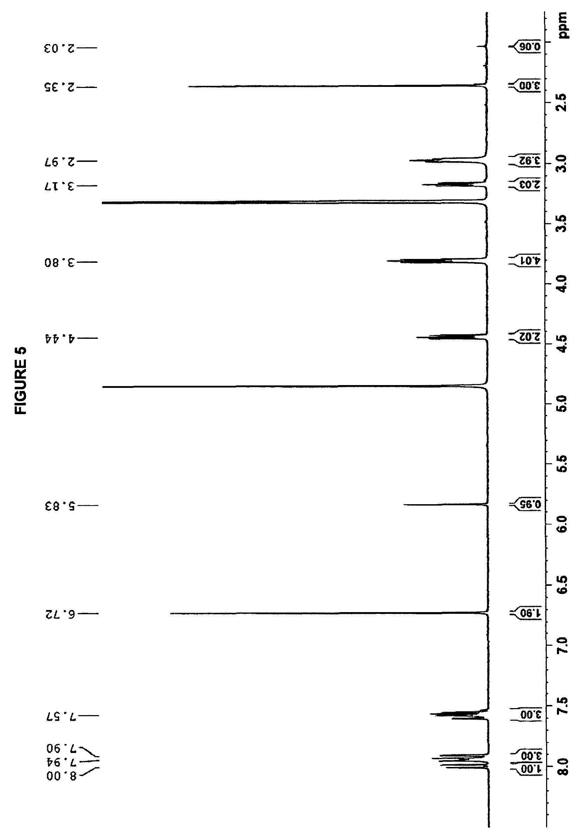

The formation of the salts can be easily characterized by the ¹H-NMR spectrum which changes substantially compared to the free base. In the case of the fumarate salt, signals coming from hydrogen atoms close to the basic nitrogen (hydrogens 1 and 2 in the formula below) are clearly shifted downfield (table 7). Smaller shifts can also be observed on signals coming from hydrogen atoms further away from the nitrogen (hydrogens 3 and 4 in the formula below). Moreover, the signal from the fumarate appears on the expected chemical shift (δ: 6.72 ppm). The integrations of signals corresponding to the anion and the cation unambiguously confirm that the equimolecular salt, and not the disalt, Is formed (Figure 5),

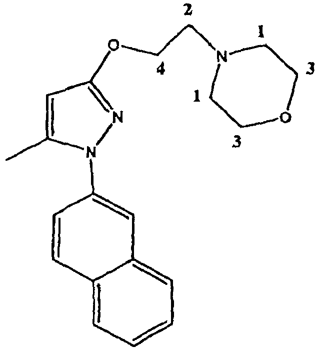

Molecular formula of compound 63 with indication of hydrogens that shift in the ¹H-NMR spectrum after forming the salt.

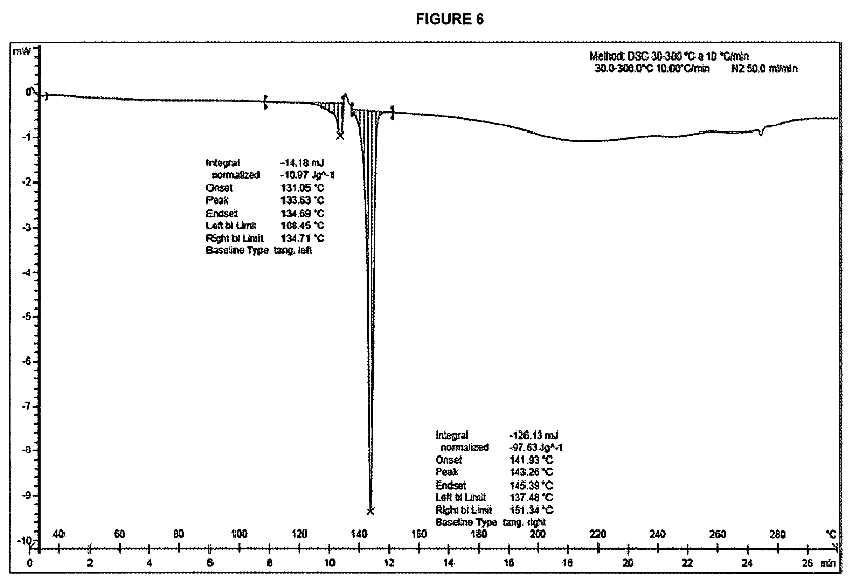

The DSC analysis at a heating rate of 10 °C/min presents a small endothermic peak, followed by a small exothermic peak and an intense endothermic signal (Figure 6). The intense signal with an onset at 142 °C corresponds to the melting temperature of solid S5. The small peak with an onset at 131 °C corresponds to the melting of the crystalline solid S3. This peak is very weak, most probably because solid S3 partially transforms to solid S5 on the heating process of the DSC analysis. Thus, the peak corresponds to the melting of the remaining S3 left at the melting temperature, which readily crystallizes to S5 (small exothermic peak). The melting peak of essentially pure solid S3 samples has different intensities depending on the specific sample. Most probably, the S3 to S5 solid-solid transition takes place to a different extend depending on the crystal habit and crystal dimensions. Therefore, samples of pure S3 crystalline solid will show DSC profiles with a shape as depicted in Figure 6.

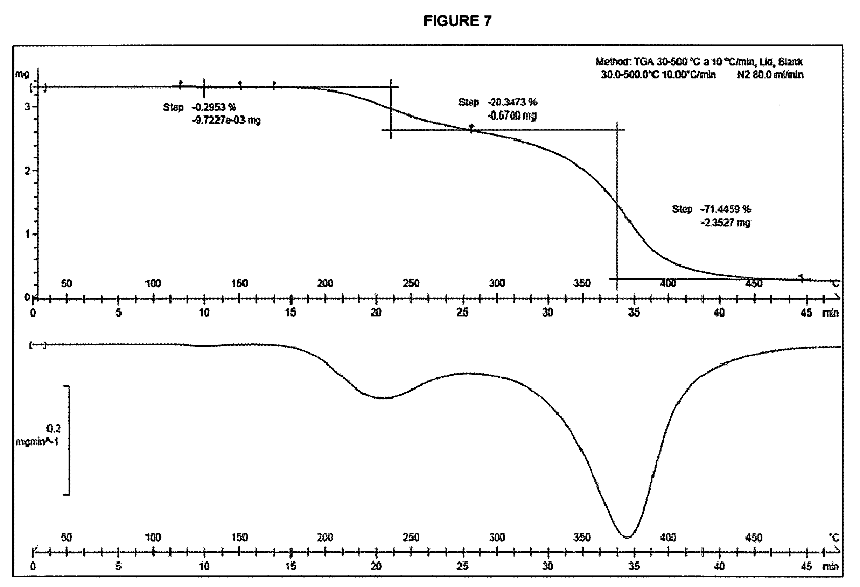

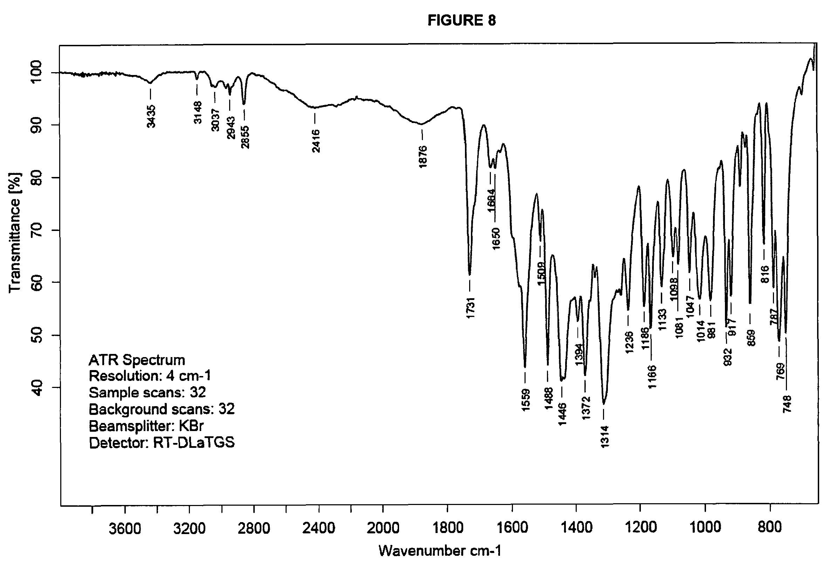

On the TG analysis a small weight loss of 0.3% at temperatures between 120 and 150 °C and a dramatic weight loss starting at 190 °C due to decomposition are observed. The characterisation of the fumarate salt is the following (Figures 5-8):
*¹H*-*NMR* (400 MHz, d4-methanol) δ: 2.35 (s, 3H), 2.92-3.00 (m, 4H), 3.17 (t, *J* = 5 Hz, 2H), 3.80 (t, *J* = 5 Hz, 4H), 4.44 (t, *J* = 5 Hz, 2H), 5.83 (s, 1 H), 6.72 (s, 2H), 7.52-7.62 (m, 3H), 7.89-7.96 (m, 3H), 8.00 (d, *J* = 9 Hz, 1 H).
*Residual solvents* from ¹H-NMR: 0.2 % w/w of acetonitrile.
*FTIR (ATR)* u: 3435, 3148, 3037, 2943, 2855, 1876, 1731, 1664, 1650, 1559, 1509, 1488, 1446, 1394, 1372, 1314, 1236, 1186, 1166, 1133, 1098, 1081, 1047, 1014, 981, 932, 917, 859, 816, 787, 769 and 748 cm⁻¹.
*DSC* (10 °C/min): Two endothermic fusion peaks with an onset at 131 and 142 °C. *TGA* (10 °C/min): A weight loss of 0.3% between 120 and 150 °C. The decomposition process starts at 190 °C.

### Example 3: (reference) preparation of the maleate salt of compound 63

During the initial screening the crystallization of the maleate salt was attempted in 10 different solvents. The salt was very soluble in all the solvents assayed. Solubilities between 50 and 200 mg/mL were observed, except for ethyl acetate, in which the salt had a solubility of 20 mg/mL. Crystalline solids were obtained in all solvents after cooling the solution to room temperature or, for chloroform, methanol and DMF, after complete evaporation of the solvent. Four different solids were detected. A non solvated crystalline phase was obtained in the majority of the crystallizations. Moreover, a solvate was generated in THF and two other not completely characterized solids were generated in three of the experiments.

Taking into account the boiling point and the amount of solvent needed for the crystallization (66 mg/mL), isopropanol was the solvent chosen for the scale-up and synthesis of the crystalline salt. An initial attempt cooling a mixture of maleic acid and compound 63 in isopropanol from 60 °C to room temperature rendered the salt as oil (Table 7). This oil crystallized after stirring again the mixture at 60 °C for several hours. A similar methodology in more diluted conditions rendered the salt directly as a solid. Finally, the process was optimized generating the direct precipitation of the salt after adding an isopropanol solution of the acid over an isopropanol solution of compound 63 at room temperature.

**Table 7. Scale-up of the maleate salt of compound 63**

| **Scale¹** | **Isopropanol volume** | **Addition temperature** | **Yield (%)** | **Observations** |
|---|---|---|---|---|
| 200 mg | 1.5 | 60 °C | 73 | Separation of the salt as an oil |
| 200 mg | 2.0 | 70 °C | 77 | Crystallization of the salt on cooling |
| 500 mg | 6.0 | 20-25 °C | 86 | - |
| 2.5 g | 30.0 | 20-25 °C | 96 | - |

| | | | | |
|---|---|---|---|---|
| ¹ Refered to starting example 1. | | | | |

The experimental procedure used to prepare the maleate salt at 2.5 g scale was as follows:
*A solution of maleic acid (772 mg, 6.65 mmol) in 15 mL of isopropanol is added slowly to a solution of compound 63 (2.26 g, 6.69 mmol) in 15 mL of isopropanol at room temperature. An abundant white solid precipitates readily. The resulting suspension is stirred at room temperature for 2 days and it is filtered. The solid obtained is washed with isopropanol and dried under vacuum (10 mm Hg) at 45 °C for 10 hours, at 55 °C for 6 hours and at 70 °C for 17 hours to give the maleate salt as a white solid (2.82 g, 96%; contains 1.1 % of isopropanol as deduced from the ¹H*-*NMR*).

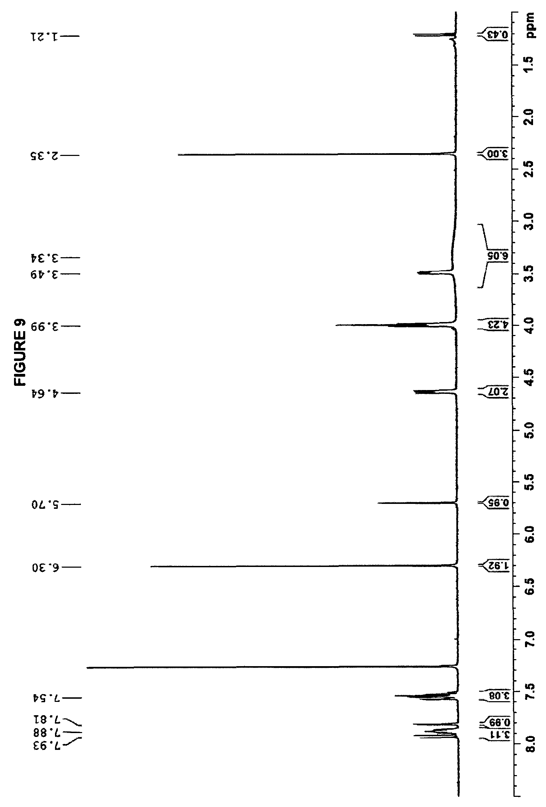

The maleate salt can be easily characterized by the ¹H-NMR spectrum (Figure 9) which changes in the same manner as has been described in depth for the fumarate salt. Moreover, the signal from the maleate appears on the expected chemical shift of 6.30 ppm. The integrations of signals corresponding to the anion and the cation unambiguously confirm that the equimolecular salt, and not the disalt, is formed.

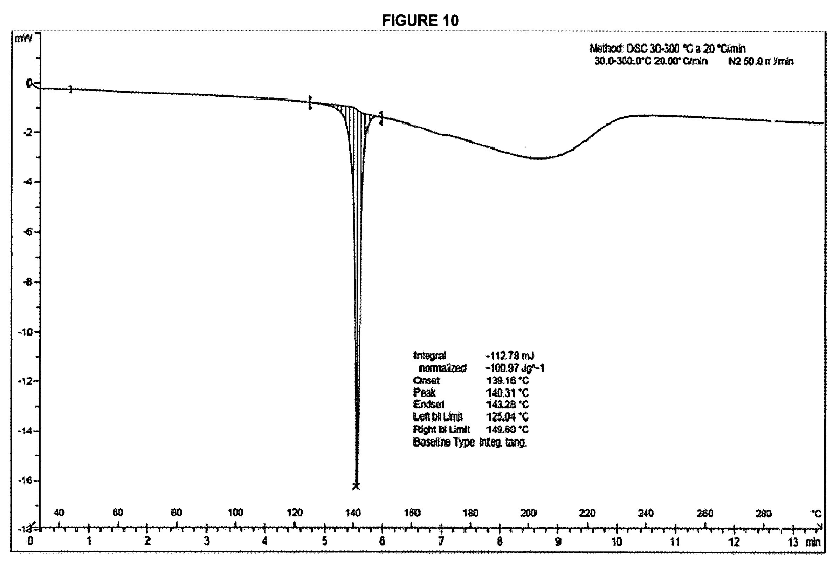

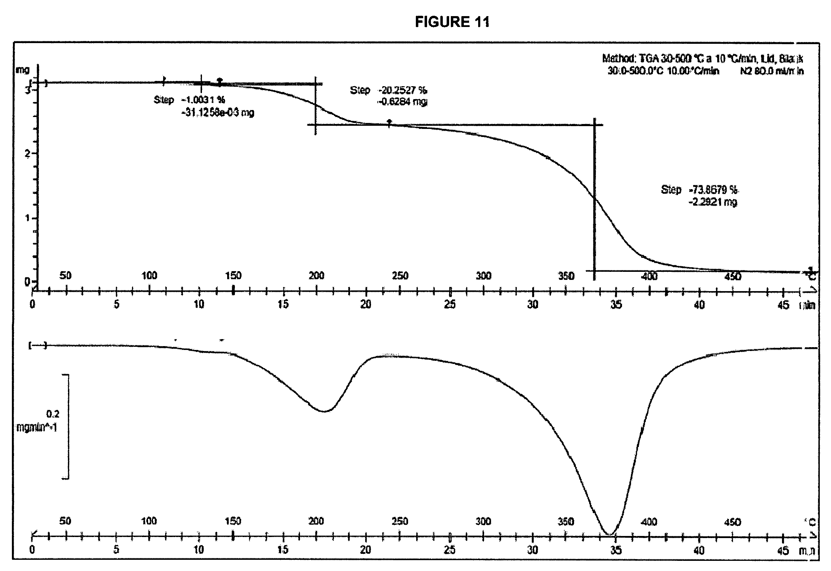

The DSC analysis (Figure 10), with a heating rate of 10 °C/min, shows an endothermic intense peak with an onset at 139 °C (101 J/g) corresponding to the melting point. A weight loss of 1 % is observed in the TGA (Figure 11) around the melting temperature, probably due to loss of residual isopropanol. Clear decomposition of the salt is observed at temperatures above 150 °C.

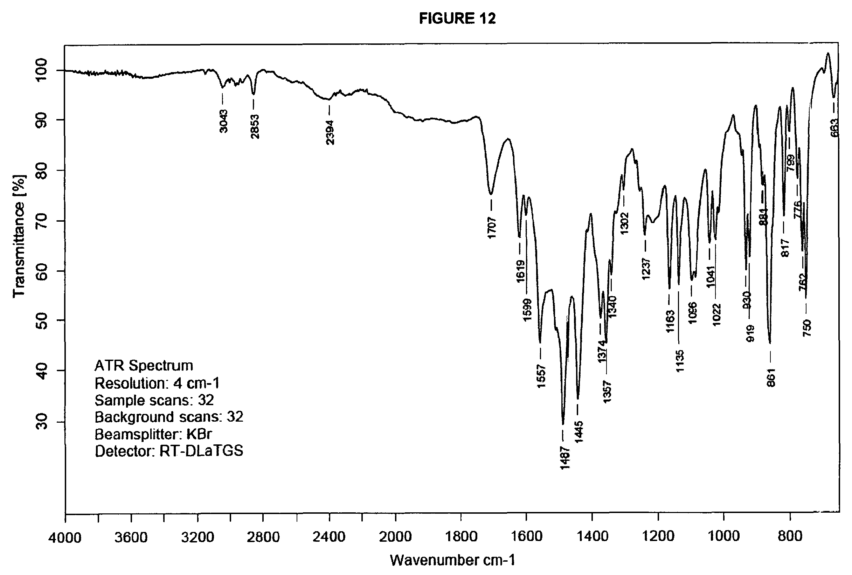

The characterisation of the maleate salt is the following (Figures 9-12):
*¹H-NMR* (400 MHz, *d*-chloroform) δ: 2.35 (s, 3H), 3.02-3.64 (m, 6H), 3.99 (t, *J* = 5 Hz, 4H), 4.61-4.66 (m, 2H), 5.70 (s, 1 H), 6.30 (s, 2H), 7.50-7.58 (m, 3H), 7.79-7.82 (m, 1 H), 7.84-7.95 (m, 3H).
*Residual solvents* from ¹H-NMR: 1.1 % w/w of isopropanol.
*FTIR (ATR)* u: 3043, 2853, 1707, 1619, 1599, 1557, 1487, 1445 1374, 1357, 1340, 1302, 1237, 1163, 1135, 1096, 1041, 1022, 930, 919, 861, 817, 762 and 750 cm⁻¹. *DSC* (10 °C/min): Endothermic fusion peak with an onset at 139 °C.
*TGA* (10 °C/min): A weight loss of 1.0% between 110-150 °C. The decomposition process starts at 150 °C.

### Example 4: (reference) preparation of the methanesulfonate salt of compound 63

During the initial screening with the first set of ten solvents, the methanesulfonate salt could not be crystallized. The salt was very soluble in all the solvents assayed (>200 mg/mL), rendering oils after complete evaporation of the solvent. When the crystallization was attempted in the second set of nine more apolar solvents, oils were also recovered in the vast majority of the experiments, either after evaporation of the solvent, or because the oily salt did not dissolve. Nevertheless, a crystalline solid corresponding to the salt was obtained from the toluene solution cooled at -18 °C after separating the excess of salt as oil. Thus, toluene was chosen for the optimization and scale-up of the synthesis of the salt.

In the first scale-up attempt, methanesulfonic acid was added directly to a toluene solution of compound 63, but the salt rapidly separated as an oil. This oil crystallized after being stirred together with the solvent for several hours at room temperature. In order to provoke the direct crystallization of the solid salt, the same process was repeated in the presence of seed crystals of the salt. Moreover, in order to improve the salt colour, the methanesulfonic acid was distilled just before use (180 °C, 1 mBar).

The experimental procedure used to prepare the methanesulfonate salt at 2.5 g scale was as follows:
*Methanesulfonic acid (0.45 mL, 6.94 mmol) is added slowly to a solution of compound 63 (2.36 g, 6.98 mmol) in 25 mL of toluene at room temperature in the presence of seeds. An abundant white solid precipitates readily. The resulting suspension is stirred at 0 °C for 8 hours and it is filtered. The solid obtained is washed with toluene and dried under vacuum (10 mm Hg) at 45 °C for 2 days and at 55 °C for 6 hours to give the methanesulfonate salt as a white solid (2.85 g, 98%; contains 0.6% of toluene as deduced from the ¹H*-*NMR*).

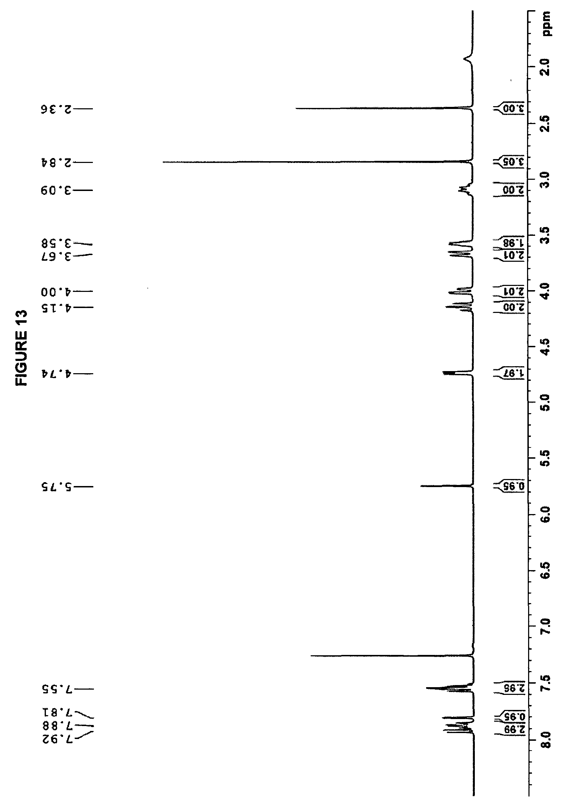

The methanesulfonate salt can be easily characterized by the ¹H-NMR spectrum (Figure 13) which changes in the same manner as has been described in depth for the fumarate salt. Moreover, the signal from the methanesulfonate appears at a chemical shift of 2.84 ppm.

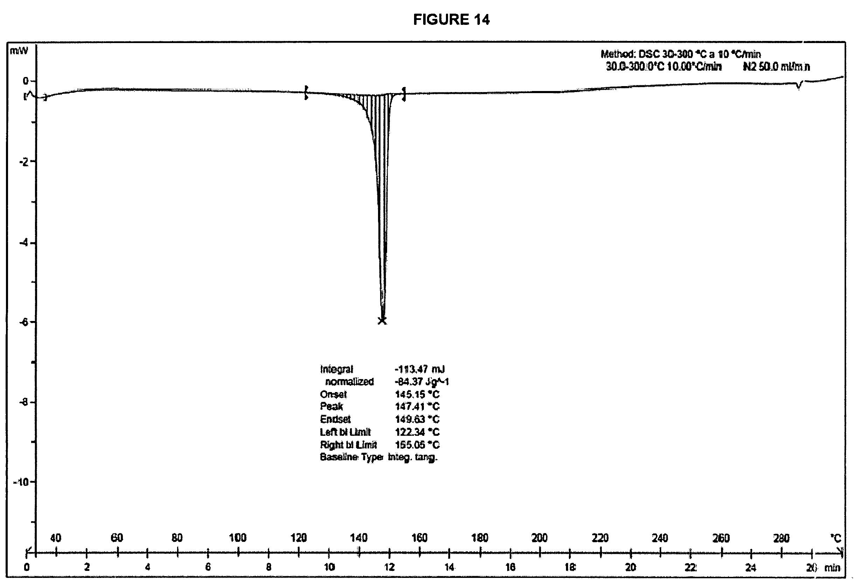

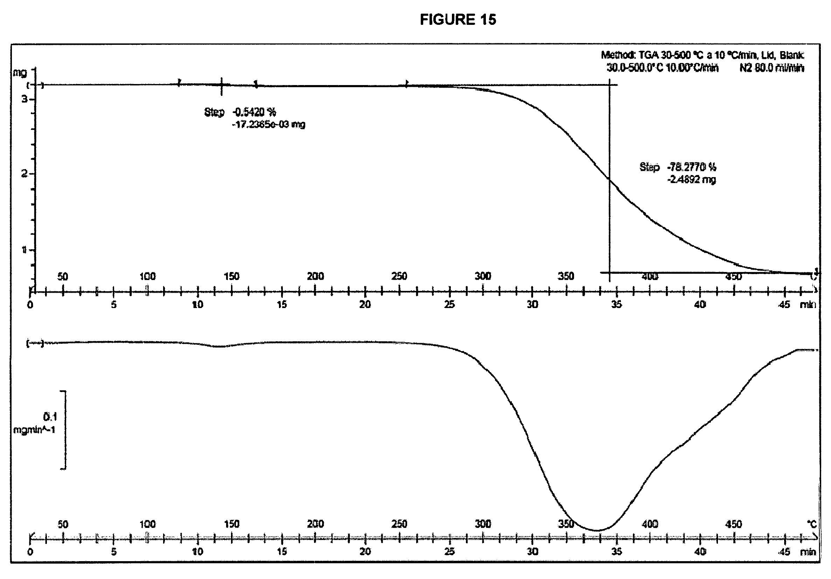

The DSC analysis (Figure 14), with a heating rate of 10 °C/min, shows an endothermic intense peak with an onset at 145 °C (84 J/g) corresponding to the melting point. A weight loss of 0.5 % is observed in the TGA (Figure 15) around the melting temperature, probably due to loss of residual toluene. Clear decomposition of the salt is observed at temperatures above 250 °C.

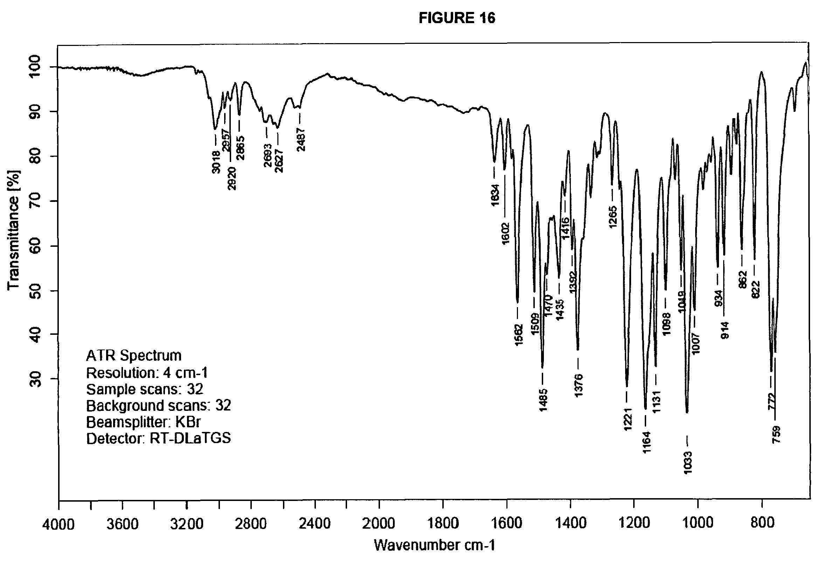

The characterisation of the methanesulfonate salt is the following (Figures 13-16):
*¹H-NMR* (400 MHz, d-chloroform) δ: 2.36 (s, 3H), 2.84 (s, 3H), 3.03-3.15 (m, 2H), 3.54-3.61 (m, 2H), 3.63-3.71 (m, 2H), 3.97-4.05 (m, 2H), 4.10-4.20 (m, 2H), 4.71-4.76 (m, 2H), 5.75 (s, 1 H), 7.50-7.59 (m, 3H), 7.79-7.82 (m, 1 H), 7.84-7.95 (m, 3H).
*Residual solvents* from ¹H-NMR: 0.58 % w/w of toluene.
*FTIR (ATR)* u: 3018, 2957, 2920, 2865, 2693, 2627, 1634, 1602, 1562, 1509, 1485, 1435, 1392, 1376, 1265, 1221, 1164, 1131, 1098, 1049, 1033, 1007, 934, 914, 862, 822, 772 and 759 cm⁻¹.
*DSC* (10 °C/min): Endothermic fusion peak with an onset at 145 °C.
*TGA* (10 °C/min): A weight loss of 0.5% between 120 and 160 °C. The decomposition process starts at 260 °C.

### Example 5: (reference) preparation of the ethanesulfonate salt of compound 63

During the initial screening with the first set of ten solvents, the ethanesulfonate salt could only be crystallized in acetonitrile. But, since the salt was very soluble in all the solvents assayed (>200 mg/mL) this solid was obtained only after complete evaporation of the solvent. In the remaining experiments, oil was generated after complete evaporation of the solvent. When the crystallization was attempted in the second set of nine more apolar solvents, three solids where obtained in methyl *tert-*butyl ether, isobutyl acetate, and toluene mixed with oily salt. In these experiments, the oily salt did not completely dissolve. Toluene was chosen to optimize and scale-up the synthesis of the salt.

In the initial scale up of the ethanesulfonate, the oily salt was suspended in hot toluene and allowed to cool. The salt did not crystallize and it remained as oil. In a second attempt, in which the ethanesulfonic acid was slowly added to a solution of compound 63 in toluene, a brown solid separated on cooling. When repeating this same procedure at room temperature, oil readily appeared which slowly crystallized after being stirred together with the solvent for several days. In order to provoke the direct crystallization of the salt, the same process was repeated at room temperature in the presence of seed crystals of the salt. Moreover, in order to improve the salt colour, the ethanesulfonic acid was distilled just before use (200 °C, 1 mBar).

The experimental procedure used to prepare the ethanesulfonate salt at 2.5 g scale was as follows:
*Ethanesulfonic acid (0.58 mL, 6.79 mmol) is added slowly to a solution of compound 63 (2.29 g*, *6.79 mmol) in 40 mL of toluene at room temperature in the presence of seeds. An abundant white solid precipitates readily. The resulting suspension is stirred at 0 °C for 12 hours and it is filtered. The solid obtained is washed with toluene and dried under vacuum (10 mm Hg) at 45 °C for 8 hours and at 55 °C for 6 hours to give the ethanesulfonate salt as a white solid (2.90 g, 99%).*

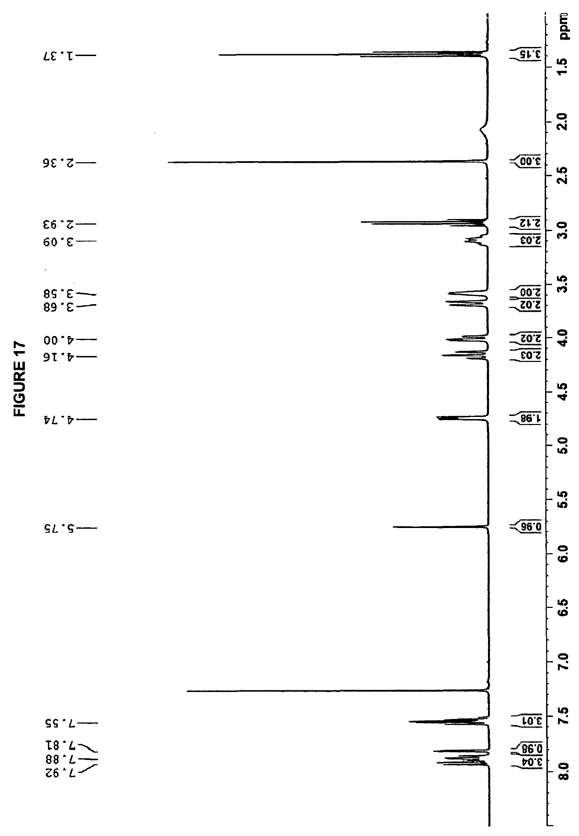

The formation of the ethanesulfonate salt can be easily deduced from the ¹H-NMR spectrum (figure 17) which changes, compared to the starting compound 63, in the same manner as has been described in depth for the fumarate salt. Moreover, signals from the ethanesulfonate appear at a chemical shift of 1.37 and 2.93 ppm.

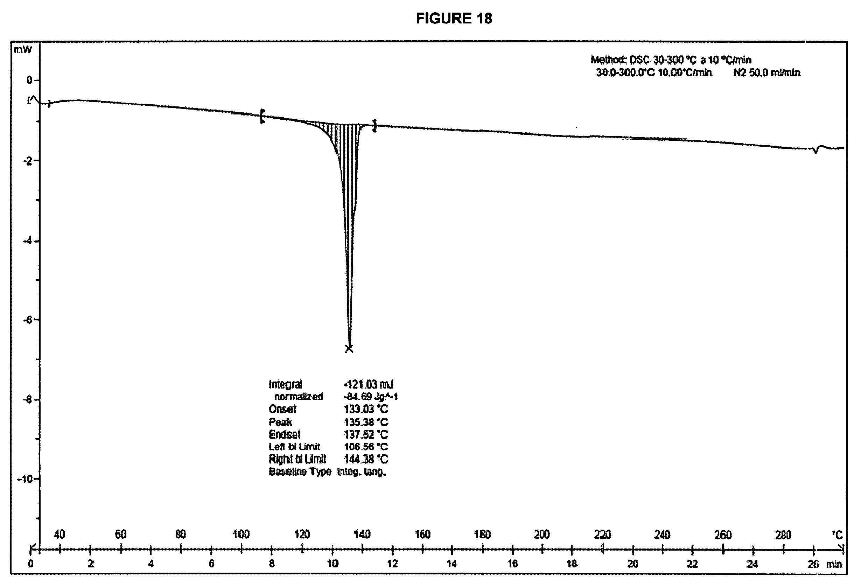

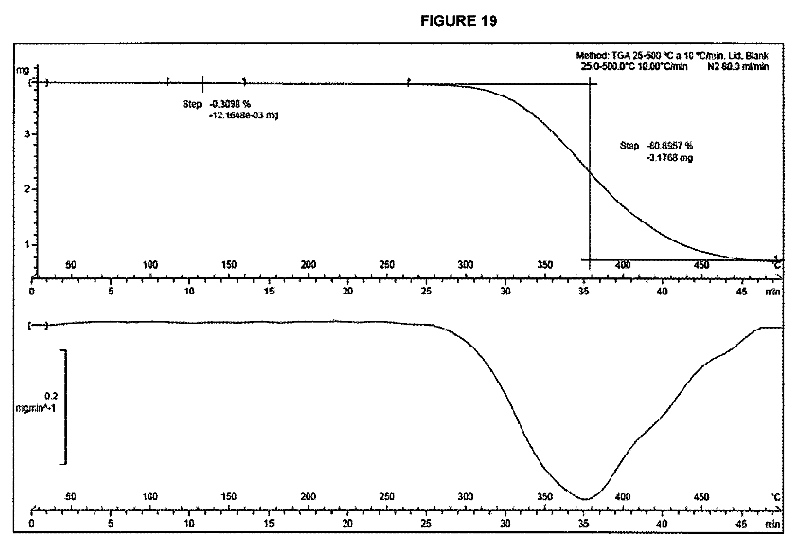

The DSC analysis (Figure 18), with a heating rate of 10 °C/min, shows an endothermic intense peak with an onset at 133 °C (85 J/g) corresponding to the melting point. A weight loss of 0.3 % is observed in the TGA (Figure 19) around the melting temperature, probably due to loss of residual toluene. Clear decomposition of the salt is observed at temperatures above 280 °C.

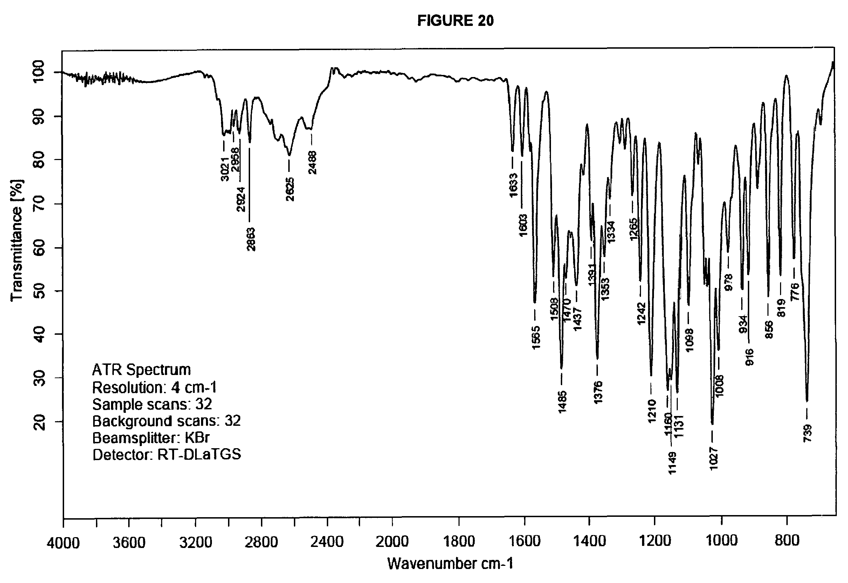

The characterisation of the ethanesulfonate salt is the following (Figures 17-20):
*¹H-NMR* (400 MHz, d-chloroform) δ: 1.37 (t, *J* = 7 Hz, 3H), 2.36 (s, 3H), 2.93 (q, *J* = 7 Hz, 2H), 3.03-3.15 (m, 2H), 3.55-3.62 (m, 2H), 3.64-3.72 (m, 2H), 3.96-4.04 (m, 2H), 4.11-4.21 (m, 2H), 4.71-4.77 (m, 2H), 5.75 (s, 1H), 7.50-7.59 (m, 3H), 7.79-7.83 (m, 1 H), 7.84-7.95 (m, 3H).
*Residual solvents* from ¹H-NMR: 0.35 % w/w of toluene.
*FTIR (ATR)* u: 3021, 2958, 2924, 2863, 2625, 2488, 1633, 1603, 1565, 1508, 1485, 1470, 1437, 1391, 1376, 1353, 1334, 1265, 1242, 1210, 1160, 1149, 1131, 1098, 1027, 1008, 978, 934, 916, 856, 819, 776, and 739 cm⁻¹.
*DSC* (10 °C/min): Endothermic fusion peak with an onset at 133 °C.
*TGA* (10 °C/min): A weight loss of 0.3% between 110 and 160 °C. The decomposition process starts at 280 °C.

### Example 6: (reference) preparation of the malate salt of compound 63

During the initial screening with the first set of ten solvents, the malate salt could be crystallized in acetonitrile and isopropanol. Nevertheless, the salt was very soluble in both solvents (>200 mg/mL) and the two solids were obtained only after complete evaporation. In the remaining experiments, oil was generated after complete evaporation of the solvent. When the crystallization was attempted in the second set of nine more apolar solvents, although the salt was less soluble, a crystalline solid was obtained only in 3-pentanone. The other experiments rendered oil. Taking into account these results, 3-pentanone was chosen to optimize and scale-up the synthesis of the salt.

The initial scale-up attempts for the preparation of the salt were performed adding a solution of L-malic acid in 3-pentanone to a solution of compound 63 also in 3-pentanone at temperatures between 50 and 70 °C. Using this procedure the salt separated sometimes as oil on cooling. This oil easily crystallized after being stirred together with the solvent at 50 °C for some hours. Direct production of the crystalline salt could be induced by seeding, as it is described in the procedure used to prepare the malate salt at 2.5 g scale that follows:
*A solution of L-malic acid (933 mg, 6.95 mmol) in 10 mL of 3-pentanone is added slowly to a solution of compound* 63 *(2.35 g, 6.95 mmol) in 10 mL of 3-pentanone at 50 °C with seed crystals. An abundant white solid precipitates readily, and the resulting suspension is diluted with another 10 mL of 3-pentanone, slowly cooled to room temperature, stirred for 12 hours and filtered. The solid obtained is washed with 3-pentanone and dried under vacuum (10 mm Hg) at 45 °C for 15 hours and at 55 °C for 6 hours to give the malate salt as a white solid (3.03 g, 95%).*

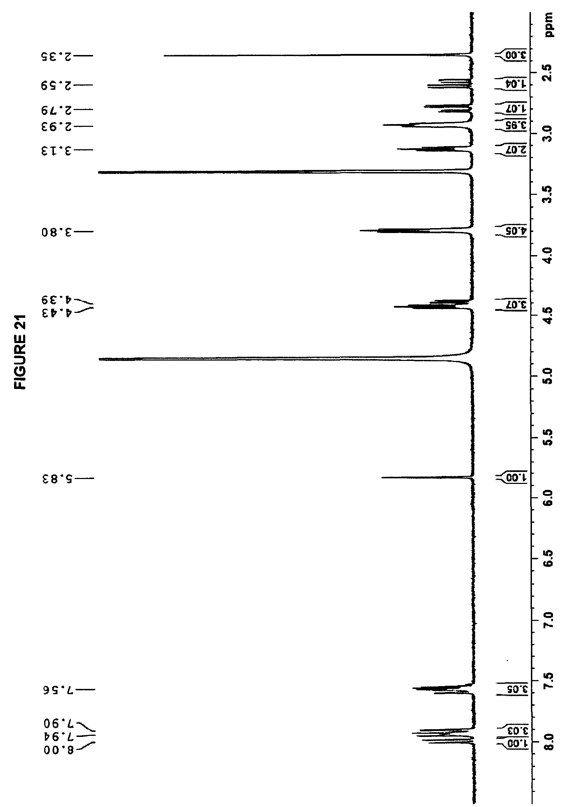

The formation of the malate salt can be easily deduced from the ¹H-NMR spectrum (figure 21) which changes significantly, compared to the starting compound compound 63, in the same manner as has been described in depth for the fumarate salt. Moreover, signals from the malate appear at a chemical shift of 2.59, 2.79 and 4.31 ppm.

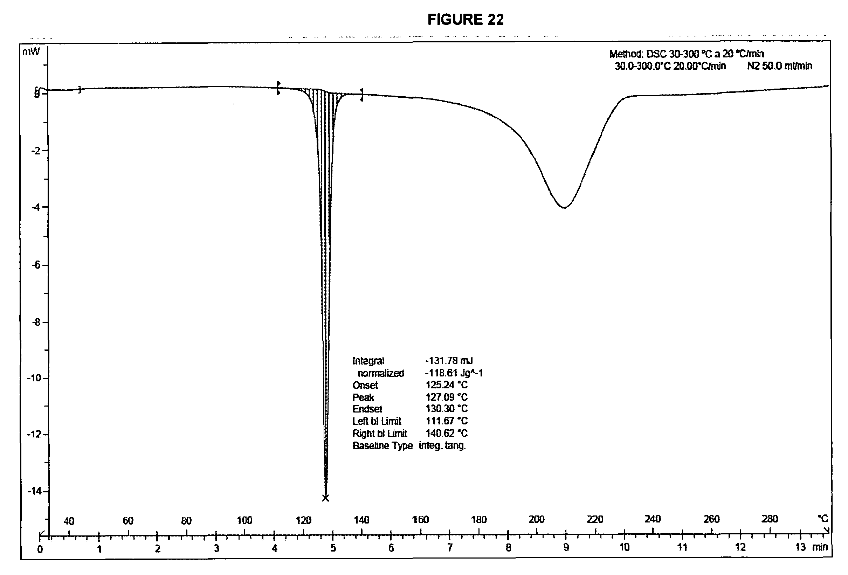

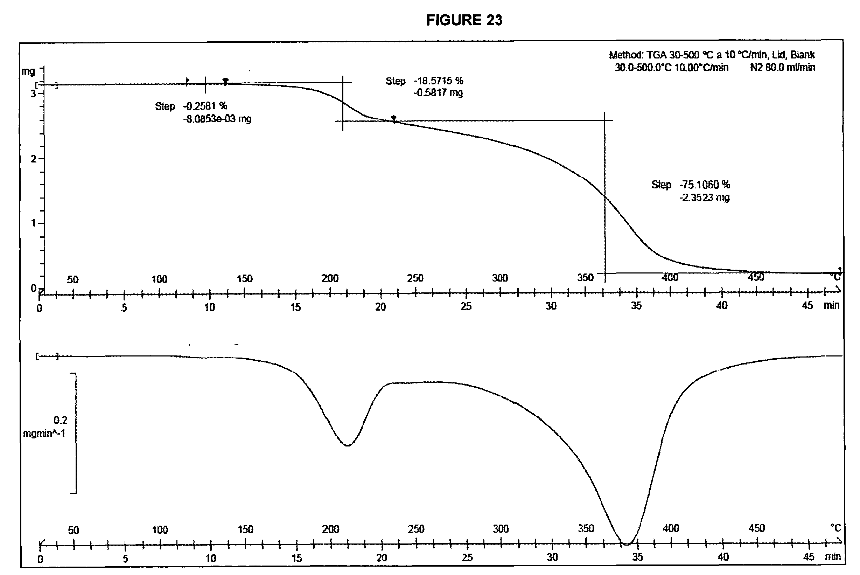

On the DSC analysis (Figure 22), with a heating rate of 10 °C/min, an endothermic intense peak with an onset at 125 °C (119 J/g) corresponding to the melting temperature is observed. Moreover, the TGA analysis (Figure 23) does not show any weight loss at temperatures below the melting point, indicating the absence of volatiles. The absence of residual solvents can also be confirmed from the ¹H-NMR spectrum.

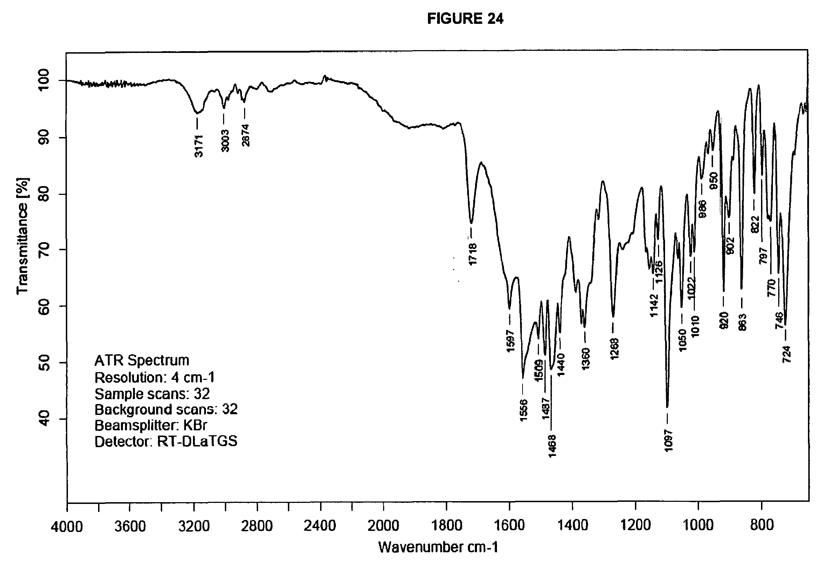

The characterisation of the malate salt is the following (Figures 21-24):
*¹H-NMR* (400 MHz, d4-methanol) δ: 2.35 (s, 3H), 2.59 (dd, *J¹* = 16 Hz, *J²* = 7 Hz, 1H), 2.79 (dd, *J¹* = 16 Hz, *J³* = 5 Hz, 1 H), 2.89-2.97 (m, 4H), 3.13 (t, *J* = 5 Hz, 2H), 3.80 (t, *J* = 5 Hz, 4H), 4.39 (dd, *J²* = 7 Hz, *J³* = 5 Hz, 1 H), 4.43 (t, *J* = 5 Hz, 2H), 5.83 (s, 1 H), 7.52-7.61 (m, 3H), 7.89-7.96 (m, 3H), 8.00 (d, *J* = 9 Hz, 1 H).
*FTIR (ATR)* u: 3171, 3003, 2874, 1718, 1597, 1556, 1487, 1468, 1440, 1360, 1268, 1142, 1126, 1097, 1050, 1022, 1010, 986, 950, 920, 902, 863, 822, 797, 770, 746 and 742 cm⁻¹.
*DSC* (10 °C/min): Endothermic fusion peak with an onset at 125 °C.
*TGA* (10 °C/min): A weight loss starting at 150 °C due to decomposition.

### Example 7: (reference) preparation of the malonate salt of compound 63

During the initial screening with the first set of ten solvents, the malonate salt could only be crystallized in isopropanol. Nevertheless, the salt was very soluble in this solvent (>200 mg/mL) which anticipated problems on scaling-up. For this reason, the crystallization was attempted in the second set of nine more apolar solvents. In this second set of experiments, a crystalline solid was obtained only from methyl *tert-butyl* ether on cooling a saturated solution to -18 °C after separating, at high temperature, an abundant part of the salt as oil.

Taking into account these results, the scale-up of the malonate salt was first attempted in isopropanol. Very disappointingly, the oil separated right after mixing the acid and compound 63. The oil crystallized in a poor yield after being stirred for several hours together with the solvent. Yield could be improved when methyl *tert-butyl* ether was added during the crystallization process after the oiling out. To avoid the generation of the salt initially as oil and to improve the yield, the crystallization process was modified. A solution of malonic acid in isopropanol was added to a solution of compound 63 in methyl *tert-butyl* ether. Using this procedure, the salt was generated directly as a solid but still some oiling out could be observed. Finally, direct and complete crystallization of the salt could be obtained with seeding as it is described in the following procedure:
*A solution of malonic acid (736 mg, 7.07 mmol) in 10 mL of isopropanol is added slowly to a solution of compound 63 (2.38 g, 7.06 mmol) in 15 mL of methyl tert-butyl ether seeded at 0 °C. An abundant white solid precipitates readily. The resulting suspension is stirred first at room temperature for 12 hours, then at 0 °C for 2 hours and it is filtered. The solid obtained is washed with methyl tert-butyl ether and dried under vacuum (10 mm Hg) at 45 °C for 7 hours and at 55 °C for 6 hours to give the malonate salt* as a *white solid (2.42 g, 80%).*

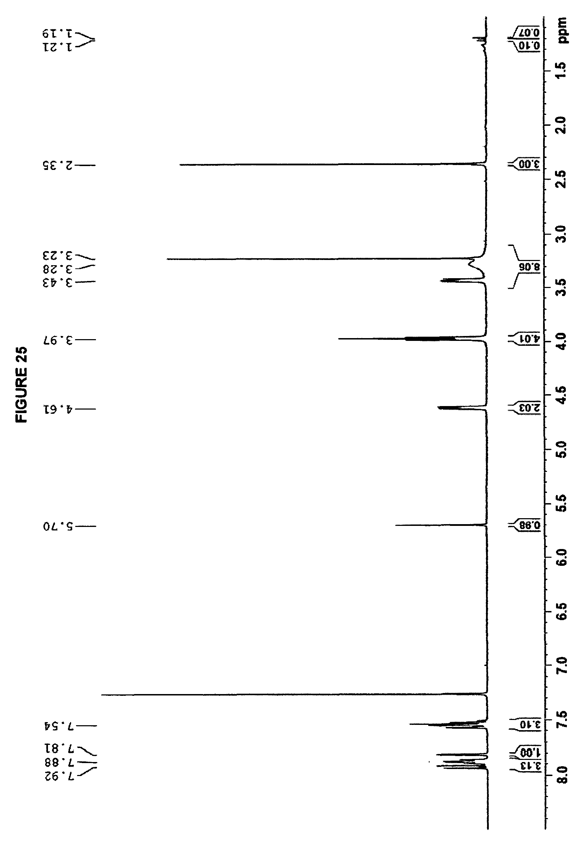

The formation of the malonate salt can be easily deduced from the ¹H-NMR spectrum (Figure 25) which changes, compared to the starting compound 63, in the same manner as has been described in depth for the fumarate salt. Moreover, signals from the malonate appear at a chemical shift of 3.23 ppm.

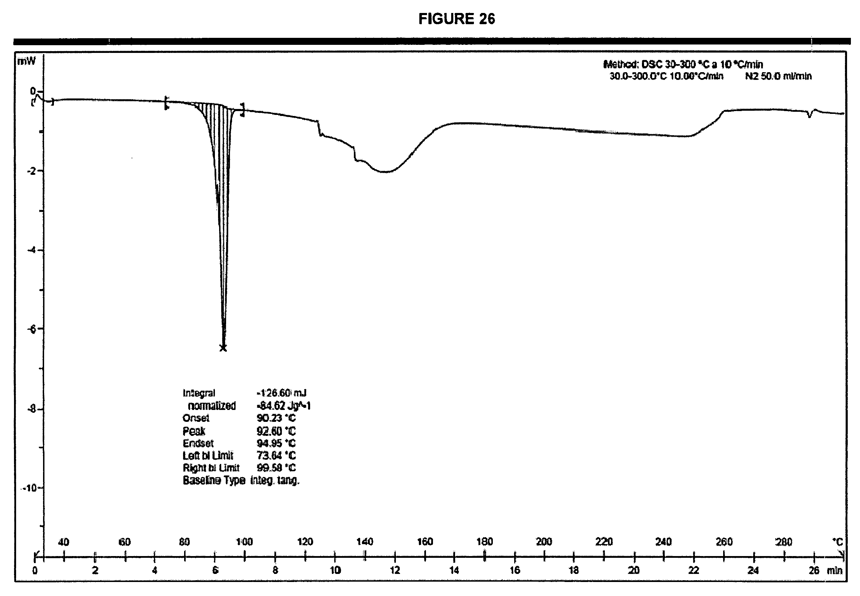

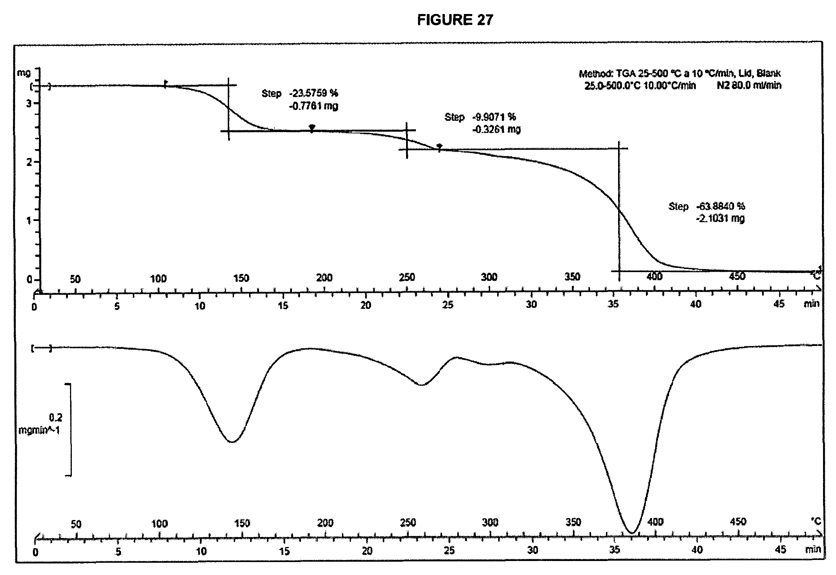

The DSC analysis (Figure 26), with a heating rate of 10 °C/min, shows an endothermic intense peak with an onset at 90 °C (85 J/g) corresponding to the melting point. Weight losses are not observed in the TGA (Figure 27) at temperatures below the melting temperature. Nevertheless, residual solvents (0.2 % w/w of isopropanol and 0.2 % methyl *tert-butyl* ether) could be detected from the ¹H-NMR spectra.

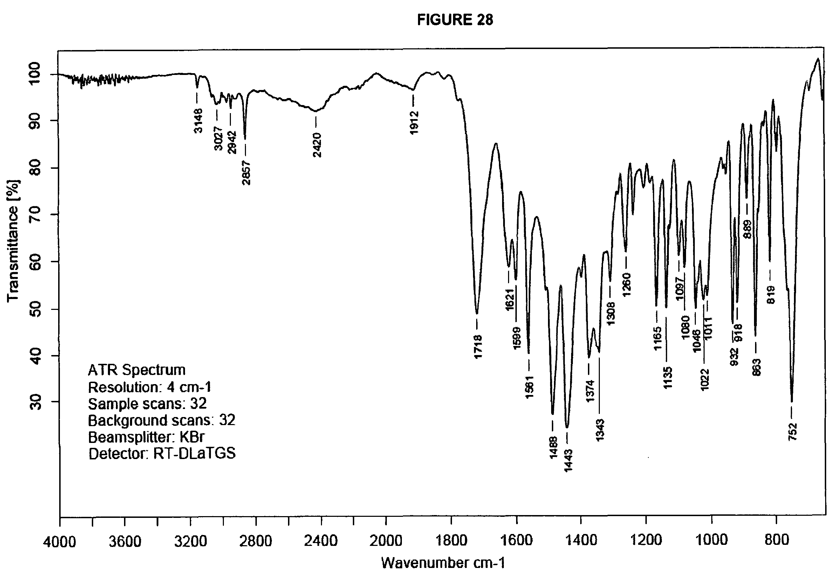

The characterization of the malonate salt is the following (Figures 25-28):
*¹H*-*NMR* (400 MHz, *d*-chloroform) δ: 2.35 (s, 3H), 3.10-3.40 (m, 4H), 3.23 (s, 2H), 3.40-3.46 (m, 2H), 3.97 (t, *J* = 5 Hz, 4H), 4.59-4.64 (m, 2H), 5.70 (s, 1 H), 7.49-7.58 (m, 3H), 7.79-7.82 (m, 1 H), 7.84-7.95 (m, 3H).
*Residual solvents* from ¹H-NMR: 0.2 % w/w of isopropanol and 0.2 % of methyl *tert-*butyl ether.
*FTIR (ATR)* u: 3148, 3027, 2942, 2857, 1718, 1621, 1599, 1561, 1488, 1443, 1374, 1343, 1308, 1260, 1165, 1135, 1097, 1080, 1046, 1022, 1011, 932, 918, 863, 819 and 752 cm⁻¹.
*DSC* (10 °C/min): Endothermic fusion peak with an onset at 90 °C.
*TGA* (10 °C/min): Weight loss starting at 100 °C due to decomposition.

### Summary of salt crystallization screening

Attempts to form salts of compound 63 with sulphuric acid and L-tartaric acid were unsuccessful and only oils were obtained.

Other salts, although in solid form, were only obtained by a complex synthetic process on comparing it with the experimental part for the hydrochloride synthesis, or under unique experimental conditions. Further, a non crystalline solid was frequently obtained instead of the crystalline form obtained for the hydrochloride. All these drawbacks imply that the scale-up for the associated synthetic process will be very complicated.

In the following table 8 a summary of key data referred to each solid salt prepared in large scale in this invention is shown: grade of crystallinity, crystallization solvent, yield and melting temperature.

**Table 8**

| **Salt** | **Crystallinity** | **Solvent / Yield** | **Melting temperature** |
|---|---|---|---|
| **Hydrochloride** | Crystalline | Isopropanol/63%* | 194°C |
| **Fumarate** | Crystalline | Ethanol / acetonitrile 59% | 131 °C |
| **Maleate** | Crystalline | isopropanol / 96 % | 139 °C |
| **Methanesulfonate** | Crystalline | toluene / 98 % | 145 °C |
| **Ethanesulfonate** | Crystalline | toluene / 99 % | 133 °C |
| **Malate** | Crystalline | 3-pentanone / 95 % | 125 °C |
| **Malonate** | Crystalline | isopropanol / methyl *tert-butyl* ether 80 % | 90 °C |

| | | | |
|---|---|---|---|
| (*) two crystallizations were made (see example 1) | | | |

As may be observed from the above, the hydrochloride salt is always obtained as a crystalline solid with a very good yield (including crystallization) and has a melting point over 50°C among the other salts which clearly implies an advantage relating to the physical stability. Additionally, on comparing the TGA analysis the hydrochloride has a clean profile and no solvent loses are detected.

Further, some additional experiments (thermodynamic solubility, pharmacokinetic) were performed for example 1 (P027) in order to confirm the suitability of this compound for pharmaceutical purposes.

### Example 8 - Thermodynamic Solubility

General protocol for thermodynamic solubility at pH 7.4 and pH 2 is described below.

### o A) Thermodynamic Solubility at pH 7.4

### Buffer pH 7.4 (50mM)

Buffer phosphates pH 7.4 was prepared as follows:
- A solution 25 mM of Na₂HPO₄.12H₂O (for 1 I of water, weight 8.96 g) was prepared
- A solution 25 mM de KH₂PO₄ (for 1 I of water weight 3.4 g) was prepared.
- 812 ml of disodium phosphate solution + 182 ml of potassium phosphate solution were mixed and pH checked according was 7.4.

### Samples equilibrium

Samples were equilibrated using:
- Stirrer Thermomixer Control of Eppendorf a 25°C y 1250 rpm
- pHmeter with combined electrode of pH semimicro

### Procedure

### Problem compound

2 mg in an HPLC vial (by duplicate) was weight and 1 ml of buffer was added. The vial was maintained at 25°C, in the stirrer Thermomixer Comfort., during 24 hours. Centrifugation at 4000 rpm followed during 15 min.

The resulting upper layer was collected with a glass pipette and transferred to the HPLC vials. Again centrifuged and the injector programmed at 2.7 mm high.

### Standards (by duplicate)

Sol.A: 2 mg in 5 ml methanol (400 ug/ml)
Sol.B: 1 ml Sol.A to 10 ml with methanol (40 ug/ml)
Sol.C: 5 ml Sol.B to 50 ml with methanol (4 ug/ml)
Sol.D: 4 ml Sol.C to 10 ml with methanol (1.6 ug/ml)
Sol.E: 5 ml Sol.D to 25 ml with methanol (0.32 ug/ml)

10 µl of all prepared solutions were injected, beginning with the more diluted standard. Blanks were also injected, for checking the absence of contamination.

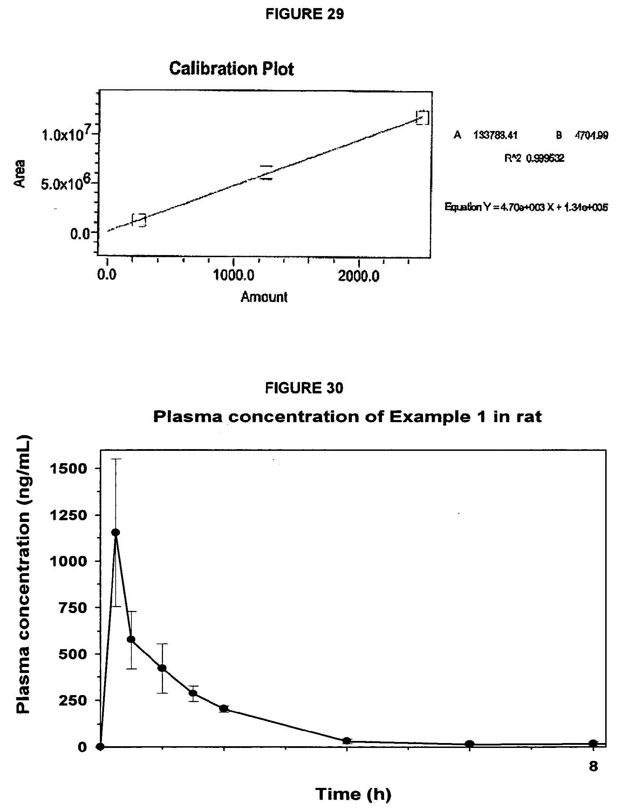

The standard calibration curve was done (see Figure 29). Consider Y = area y X = µg injected standard

10 µl of problem compound solution were injected, by duplicate and the average peak area (if quantifiable) interpolated in the standard curve (see Tables 9, 10 and 11 and example below).

### Chromatographic conditions

- Column: XBridge C18 (or similar) 2.5 µm 4.6x 50 mm
- Temperature: 35°C
- Mobile phase: ACN / ammonium bicarbonate 10 mM.
- Gradient:
   0-3.5 min: from 15% ACN to 95% ACN
   3.5 - 5 min: 95% ACN
   5 - 6 min: 95 a 15% ACN
   6 - 8 min: 15% ACN
- Flow: 1.5 ml/min
- Detection: around the UV absorption maximum

### o B) Thermodynamic Solubility at pH 2

The previous procedure was executed with HCl 0.01 N.

### Thermodinamical solubility for example 1

According to the described protocol it was obtained 227 *µg*/*ml* (pH=7.4). See associated graphic in Figure 29.

**Table 9**

| **CALIBRATION** | | | | | | | | | | | | |
|---|---|---|---|---|---|---|---|---|---|---|---|---|
| **Peak: Muestra** | | | | | | | | | | | | |
| SampleName | Date Acquired | Vial | RT (min) | Vol. (ul) | Detection | Dil | X Value | Area | Res. Id | Cal Id | S.Weight | Height (uV) |
| Example 1 Pat.50ug/ml) 1 | 22/07/2010 17: 09:51 | 3 | 16.1 | 5 | PDA 260.0 nm | 100.00 | 250.000 | 1235989 | 40781 | 40782 | 5000.000 | 225760 |
| Example 1 Pat.(50ug/ml) 1 | 22/07/2010 17:40:38 | 3 | 16.1 | 5 | PDA 260.0 nm | 100.00 | 250.000 | 1237942 | 40785 | 40782 | 5000.000 | 226564 |
| Example 1 Pat.(250ug/ml) 1 | 22/07/2010 18:11:31 | 4 | 16.1 | 5 | PDA260.0 nm | 20.00 | 1250.000 | 6158085 | 40787 | 40782 | 5000.000 | 1132809 |
| Example 1 Pat.(250ug/ml) 1 | 22/07/2010 18:42:21 | 4 | 16.1 | 5 | PDA 260.0 nm | 20.00 | 1250.000 | 6135000 | 40789 | 40782 | 5000,000 | 1129396 |
| Example 1 Pat.(500ug/ml) 1 | 22/07/2010 19:13:10 | 5 | 16-1 | 5 | PDA 260.0 nm | 10.00 | 2500.000 | 11 826040 | 40791 | 40782 | 5000.000 | 2158910 |
| Example 1 Pat.[500ug/ml) 1 | 22/07/2010 19:44:00 | 5 | 16-1 | 5 | PDA 260.0 nm | 10.00 | 2500.000 | 11849583 | 40793 | 40782 | 5000.000 | 2168579 |

**Table 10**

| **SAMPLES** | | | | | | | | | | |
|---|---|---|---|---|---|---|---|---|---|---|
| **muestra:pH7.4** | | | | | | | | | | |
| | **Sample** | **muestra** | **Vial** | **RT** | **Date Acquired** | **Dilution** | **Inj.Vol. (ul)** | **Detection** | **Area** | **Height** |
| 1 | Example 1 PROB 1 | pH7.4 | 13 | 16.1 | 23/07/2010 14:30:00 | 1.00 | 5 | PDA 260.0 nm | 5520635 | 1006234 |
| 2 | Example 1 PROB 1 | pH7.4 | 13 | 16.1 | 23/07/2010 15:00:50 | 1.00 | 5 | PDA 260.0 nm | 5527190 | 1002480 |
| 3 | Example 1 PROB 2 | pH7.4 | 14 | 16.1 | 23/07/2010 15:31:42 | 1.00 | 5 | PDA 260.0 nm | 5433650 | 992252 |
| 4 | Example 1 PROB 2 | pH7.4 | 14 | 16.1 | 23/07/2010 16:02:29 | 1.00 | 5 | PDA 260.0 nm | 5438948 | 988427 |
| Mean | | | | | | | | | | |
| % RSD | | | | | | | | | | |

**Table 11**

| **SAMPLES** | | | | | |
|---|---|---|---|---|---|
| **muestra: pH7.4** | | | | | |
| | **Conc.** | **Units** | **Res Id** | **Cal Id** | **SampleWeight** |
| 1 | 229.0 | ug/ml | 40794 | 40782 | 1.00 |
| 2 | 229.3 | ug/ml | 40795 | 40782 | 1.00 |
| 3 | 225.3 | ug/ml. | 40796 | 40782 | 1.00 |
| 4 | 225.5 | ug/ml | 40797 | 40782 | 1.00 |
| Mean | 227.262 | | | | |
| % RSD | 0.9 | | | | |

### Example 9 - Pharmacokinetic parameters Cmax and AUC

The pharmacokinetics of Example 1 in Wistar Hannover rats following a single oral administration of 25 mg/kg (expressed as compound 63) was tested. For this purpose, plasma samples were collected at different time points and analyzed using HPLC (High pressure liquid chromatography) method with fluorescence detection.

### Sample obtention

Two groups were used in this test. Group 1 received vehicle and Group 2 received Example 1 at 25 mg/kg with an administration volume of 10 mL/kg.

Blood samples were extracted from the retro-orbital zone at the following time points: pre-dose, 15min, 30min, 1 h, 1.5h, 2h, 3h, 4h, 5h, 6h, 8h and 24h. Blood was then transferred into heparin-containing plastic tubes. Plasma was obtained by centrifugation at approximately 3000 rpm for 10 min at 4°C. These plasma samples were labeled and frozen at a temperature of approximately - 65°C until analysis.

### Analysis of samples

Samples were analyzed by a previously validated analytical method. Briefly, rat plasma samples were thawed at room temperature and centrifuged at 3000 rpm for 10 min at approximately 4°C. 300 µl of plasma samples were placed into vials and spiked with 30 µl of internal standard working solution. The vials were capped and mixed thoroughly.

The following solid-phase extraction method was used for the extraction of Example 1.
1. Cartridge activation with methanol for 1 min at 1.5 ml/min.
2. Cartridge activation with water for 2 min at 1.5 ml/min.
3. Sample loading (80 µl) in the cartridge with water for 1.5 min at 1.0 ml/min.
4. Rinsing with water/ACN (90/10, v/v) for 30 s. at 1.5 ml/min.
5. Sample elution with the mobile phase for 1 min at 0.5 ml/min.
6. Cartridge and capillary washing with water and methanol.

Samples were then chromatographied using as mobile phase a mixture of 20 mM potassium phosphate monobasic adjusted at pH 3, and acetonitrile (70-73 %) A and (30 - 27%) B (v/v) at room temperature. The flow rate used was 0.5 ml/min and analysis time was around 17 min.

The peaks corresponding to Example 1 and its internal standard were quantified by fluorescence detection at an excitation wavelength of 260 nm and an emission wavelength of 360 nm. The rest of parameters were: Response time: >0.2 min (4s standard) and PMT gain 8.

### Pharmacokinetic parameters

The pharmacokinetic parameters were obtained from the mean plasma level curves by means of non-compartmental kinetics using the software program WinNonlin Professional version 5.0.1.

The peak plasma concentration values (Cₘₐₓ) and the time to reach such concentration (tₘₐₓ) were obtained directly from the experimental data. The elimination constant (kₑₗ) was calculated by linear regression of the last phase of the curve (log concentration vs. time). The elimination half-life (t_{1/2}) was determined with the expression t_{1/2}= 0.693/kₑₗ. The area under the curve of plasma levels vs. time from zero to the last time determined (AUC₀₋ₜ) was calculated be means of the trapezoidal method. The area under the curve of plasma levels vs time from zero to infinity (AUC_{0-∞}) was calculated with the expression: AUC_{0-∞}= AUC₀₋ₜ⁺Cₗₐₛₜ/kₑₗ, where Cₗₐₛₜ is the plasma concentration at the last time measured.

### Pharmacokinetic parameters Cmax and AUC of example 1

According to the described protocol it was obtained Cmax: 1152.8 ng/ml, AUC₀₋ₜ: 1218.4 ng.h/ml and AUC_{0-∞}: 1249.6 ng.h/ml. See associated graphics in Figure 30.

The results obtained in the last two tests (solubility and pharmacokinetic) enforce the hydrochloride as the better salt for compound 63 for related formulations and clinical studies.

## Claims

1. Hydrochloride salt of 4-[2-[[5-methyl-1-(2-naphthalenyl)-1 H-pyrazol-3-yl]oxy]ethyl]morpholine.

2. A process for the preparation of the hydrochloride salt of claim 1, comprising:
a) mixing 4-[2-[[5-methyl-1-(2-naphthalenyl)-1 H-pyrazol-3-yl]oxy]ethyl]morpholine and a solution containing hydrochloric acid, and
b) isolating the resulting hydrochloride salt.

3. A pharmaceutical composition comprising the hydrochloride salt of claim 1.

4. The hydrochloride salt of claim 1 for use as medicament.

5. The hydrochloride salt of claim 1 for use in the treatment and/or prophylaxis of a sigma receptor mediated disease or condition.

6. The hydrochloride salt of claim 1 for use in the treatment and/or prophylaxis of a disease selected from the group consisting of diarrhoea; lipoprotein disorders; migraine; obesity; arthritis; hypertension; arrhythmia; ulcer; learning, memory and attention deficits; cognition disorders; neurodegenerative diseases; demyelinating diseases; addiction to drugs and chemical substances including cocaine, amphetamine, ethanol and nicotine; tardive dyskinesia; ischemic stroke; epilepsy; stroke; stress; cancer; psychotic conditions, in particular depression, anxiety or schizophrenia; inflammation; or autoimmune diseases.

## Patentansprüche

1. Hydrochloridsalz von 4-[2-[[5-Methyl-1-(2-naphthalinyl)-1 H-pyrazol-3-yl]oxy]ethyl]morpholin.

2. Verfahren zur Herstellung des Hydrochloridsalzes nach Anspruch 1, umfassend:
a) Mischen von 4-[2-[[5-Methyl-1-(2-naphthalinyl)-1 H-pyrazol-3-yl]oxy]ethyl]morpholin und einer Salzsäure enthaltenden Lösung, und
b) Abtrennen des so erhaltenen Hydrochloridsalzes.

3. Pharmazeutische Zusammensetzung, umfassend das Hydrochloridsalz nach Anspruch 1.

4. Hydrochloridsalz nach Anspruch 1 zur Verwendung als Arzneimittel.

5. Hydrochloridsalz nach Anspruch 1 zur Verwendung bei der Behandlung und/oder Prophylaxe einer durch den Sigmarezeptor vermittelten Erkrankung oder eines durch den Sigmarezeptor vermittelten Zustandes.

6. Hydrochloridsalz nach Anspruch 1 zur Verwendung bei der Behandlung und/oder Vorbeugung einer Erkrankung, ausgewählt aus der Gruppe bestehend aus Diarrhö; Lipoproteinstörungen; Migräne; Adipositas; Arthritis; Hypertonie; Arrhythmie; Ulkus; Lern-, Gedächtnis- und Aufmerksamkeitsdefiziten; Kognitionsstörungen; neurodegenerativen Erkrankungen; demyelinisierenden Erkrankungen; Abhängigkeit von Drogen und chemischen Substanzen, einschließlich Kokain, Amphetamin, Ethanol und Nikotin; tardiver Dyskinesie; ischämischem Schlaganfall; Epilepsie; Schlaganfall; Stress; Krebserkrankung; psychotischen Zuständen, insbesondere Depression, Angstzuständen oder Schizophrenie; Entzündung; oder Autoimmunerkrankungen.

## Revendications

1. Sel de chlorhydrate de la 4-[2-[[5-méthyl-1-(2-naphtalényl)-1 H-pyrazol-3-yl]oxy]éthyl]morpholine.

2. Un procédé de préparation du sel de chlorhydrate de la revendication 1, comprenant:
a) le mélange de la 4-[2-[[5-méthyl-1-(2-naphtalényl)-1 H-pyrazol-3-yl]oxy]éthyl]morpholine et d'une solution contenant de l'acide hydrochlorique, et
b) l'isolation du sel chlorhydrate ainsi obtenu.

3. Une composition pharmaceutique comprenant le sel de chlorhydrate de la revendication 1.

4. Le sel de chlorhydrate de la revendication 1 pour utilisation comme médicament.

5. Le sel de chlorhydrate de la revendication 1 pour une utilisation dans le traitement et/ou la prophylaxie d'une maladie ou d'une affection médiée par le récepteur sigma.

6. Le sel de chlorhydrate selon la revendication 1 pour une utilisation dans le traitement et/ou la prophylaxie d'une maladie sélectionnée parmi le groupe constitué de diarrhée ; troubles liés aux lipoprotéines ; migraine ; obésité ; arthrite ; hypertension ; arythmie ; ulcère ; déficits de l'apprentissage, de la mémoire et de l'attention ; troubles cognitifs ; maladies neurodégénératives ; maladies de démyélinisation , addiction aux drogues et aux substances chimiques y compris la cocaïne, les amphétamines, l'éthanol et la nicotine ; dyskinésie tardive ; accident vasculaire cérébral ischémique ; épilepsie ; accident vasculaire cérébral ; stress ; cancer ; affections psychotiques, notamment la dépression, l'anxiété, ou la schizophrénie ; inflammation ; ou maladies auto-immunes.
